# EUROPEAN PATENT APPLICATION

(11) **EP 0 978 562 A1**
(43) Date of publication of application: **09.02.2000**
(21) Application number: 98202654.4
(22) Date of filing: 06.08.1998
(51) Int. Cl.: C12N 15/12, C12N 15/54, C12N 9/12, C07K 14/47, A61K 38/17

(54) **Calcium/calmodulin dependent kinase and alternative splice product thereof**

(71) Applicant: Division of Medical Pharmacology Leiden/ Amsterdam Centre for Drug Research (LACDR), 2333 AL Leiden (NL)
(72) Inventor: The designation of the inventor has not yet been filed
(74) Representative: Smulders, Theodorus A.H.J., Ir.

(57) **Abstract**

The present invention provides novel means and methods for regulation of calcium/calmodulin dependent processes. In particular the present invention provides a novel kinase CaMK VI and a peptide involved in feedback of CAM kinases, named CARP.

## Description

The present invention relates to the field of cell biology and particularly to physiological changes occurring in cells and thus in organisms as a result of a change in calcium concentrations in the cell's environment. A group of molecules that is involved in the regulation of, or affected by fluctuations in calcium concentrations is the group of calcium/calmodulin dependent kinases. These kinases play pivotal roles in many of the cell's processes and are thus also pivotal in many disorders. One of these disorders to which this invention specifically pertains is epilepsy.

Epilepsy is characterised by transient disturbances in brain function that is manifested as episodes of unconsciousness, abnormal motor patterns and physiological or sensory disturbances and affects approximately 1% of the human population. Many factors, e.g. brain damage, altered metabolic states, drug abuse, have been shown to cause epilepsy, but it is generally accepted that all these converge into a distortion of the neuronal excitation-inhibition balance. This disbalance subsequently triggers recurrent episodes of aberrant synchronisation in specific neuronal networks, in particular in the cortical layers and in the hippocampus resulting in epileptic seizures.

A number of well-established animal model systems, including kainate-induced seizures, have been used to study the electrophysiological, biochemical and molecular biological mechanisms underlying epilepsy. Studies in these systems have implicated an aberrant calcium-activated kinase activity as the major factor for the induction and onset of epileptic seizures. First, several anti-epileptic drugs are thought to target calcium channels directly or indirectly {Stefani et al., 1996, von Wegerer et al., 1997}. Second, induction of epileptic seizures in several model systems seem to alter the expression of calcium-activated kinases. For example, the calcium/calmodulin dependent protein kinase (CaMK)-II mRNA, the cellular localisation and activity of the CaMK-II protein are changed during kindling and continuous hippocampal stimulation-induced seizures {Morimoto et al, 1997; Perlin et al., 1992; Wasterlain et al., 1992}. Third, numerous immediate early genes which are activated by calcium-dependent kinase pathways are induced or upregulated after kainate or kindling-induced seizures.{Gass et al., 1994; Gass et al., 1997; Herdegen et al., 1993; Herdegen et al., 1997; Hughes et al., 1998}. Fourth, mutations in several genes with relevance for neuronal calcium-activated kinases have been shown to cause epileptic seizures in mice (reviewed in McNamara and Puranam, 1998). For example, targeted deletion or aberrant splicing in the calcium channels and in CaMK-II genes will induce an epileptic phenotype in mice. Together, these data strongly favour a model in which calcium-activated kinase mediated signal transduction pathways affect the onset and course of epileptic seizures.

In humans only a few genes have been identified which are associated with genetically predisposed forms of epileptic seizures. Recently, mutations in a novel gene, doublecortin, have been associated with the human X-linked lissencephaly and the doublecortex syndrome {des Porte et al., 1998; Gleeson et al., 1998}. This disorder is characterised by malformation of the cerebral cortex and will produce severe mental retardation and seizures {Dobyns and Truwit, 1995}.

Doublecortin shares a high degree of amino acid sequence identity over its entire length with the amino terminus of a novel CaM kinase, called KIAA0369 {Nagase et al., 1997}, but doesn't contain a kinase domain itself. It was speculated {Gleeson et al., 1998} that the malfunction of the doublecortin protein will lead to aberrant modulation of protein kinase-mediated signal transduction pathways which might underly the morphological and behavioural epileptic phenotype. In combination with the above, these data suggest that malfunctioning of calcium-affected kinase activity is crucial for the onset and maintenance of epileptic seizures.

The present invention provides in one embodiment identification and characterisation of a cDNA which is highly induced by kainate-induced seizures. The corresponding transcript is generated by alternative splicing of a novel CaMK gene, another embodiment of the present invention. The cDNA encodes a 55 amino acid peptide, called CaMK-related peptide (CARP) and plays a role in kinase-mediated neuronal plasticity which is associated with the course of epilepsy.

A large part of CARP, 48 out 55 amino acids, is highly homologous with the carboxy-terminus of the recently characterised human doublecortin. Mutations in the doublecortin gene have been shown to underly the molecular mechanism leading to X-linked lissencephaly and the doublecortin syndrome {des Portes et al., 1998; Gleeson et al., 1998}. These disorders are caused by a migrational arrest of cortical neurones to their destination which lead to a malformation of the cortex and are further characterised by severe mental retardation and epileptic seizures {Dobyns and Truwit, 1995}. As doublecortin is a likely substrate for members of the MAP kinase family, it was suggested that doublecortin transduces signals which are critical for migrating neurones {Gleeson et al., 1998}. The homology of CARP with doublecortin and the conservation of some of the phosphorylation sites show a similar role for CARP in the neuronal plasticity. Seizure activity has been shown to result in massive reorganisation of synaptic contacts, neurogenesis and affects neuronal networks in the hippocampus, in particular in the dentate gyrus {Buckmaster and Dudek, 1997; Mathern et al., 1996; Parent and Lowenstein, 1997; Parent et al., 1997; von Campe et al., 1997}. Our findings that CARP is specifically induced in the hippocampus after kainate-induced seizures and that this induction is most pronounced in the dentate gyrus, is well in line with this function of CARP.

Seizure-induced CARP expression may act as a substrate for hyperactivated kinases, although the invention is not limited to any possible mechanism of action of CARP. At the protein level, CARP overlaps with CaMK-VI at the extended, serine-rich N-terminus and has no overlap or homology with the catalytic or regulatory domain. This implies a function of CARP which is directly linked to that of the serine rich N-terminus of CaMK-VI. In this respect, the structural similarity of CaMK-VI to CaMK-IV is of interest. In contrast to other CaMK's both the rat α- and β-CaMK-IV also contain an extended, serine-rich N-terminus {Sakagami and Kondo, 1993; Means et al., 1991}. Full activation of CaMK-IV involves at least three steps: firstly, calcium/calmodulin binding will lead to exposure of the catalytic domain to a CaMK-IV kinase; secondly, phosphorylation of Thr214 induces kinase activity and thirdly, auto-phosphorylation of several serine residues in the N-terminus occurs (for review see {Means et al., 1997}. Mutation of both Ser12 and Ser13 into alanine residues in a-CaMK-IV completely abolishes the kinase activity.

Moreover, removal of the N-terminal 20 amino acids results in a kinase with wildtype CaMK-IV properties. This indicates an auto-inhibitory function of the N-terminus which can be relieved by auto-phosphorylation {Chatila et al., 1996}. The structural resemblance of the presently invented CaMK-VI with the extended serine-rich N-terminus of CaMK-IV and the presence of several phosphorylation sites in both CARP and CaMK-VI suggests a similar, 3-step activation mechanism for CaMK-VI, implying that phosphorylation of N-terminal located serine residues is crucial for full activity. Accordingly, CARP functions as a modulator of CaMK-VI and/or other kinases.

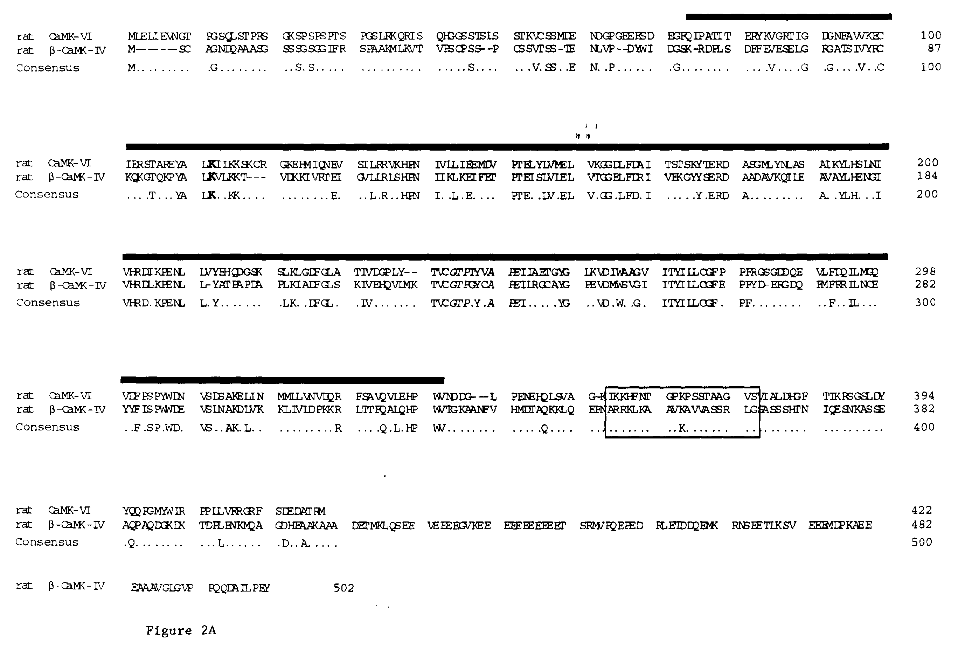

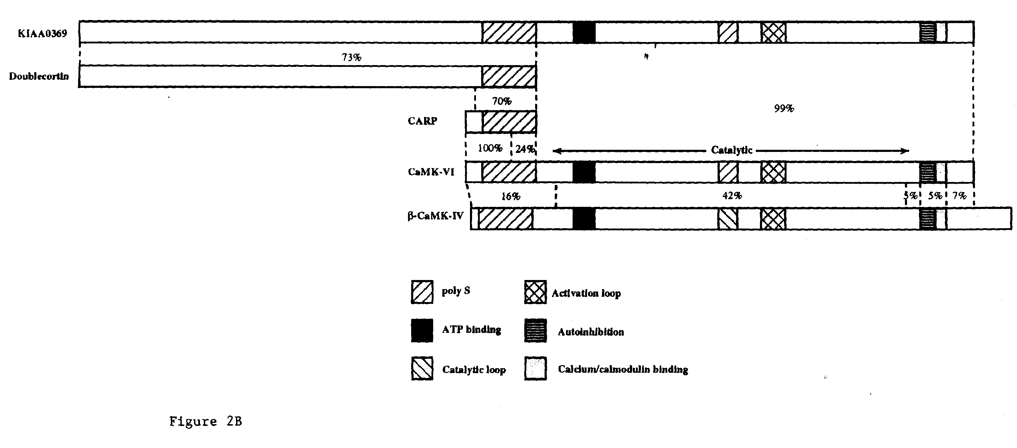

CARP is likely derived from a gene which gives rise to a number of splice variants encoding different peptides and/or proteins involved in kinase-mediated signal transduction pathways. Beside CARP, transcription of this CaMK gene leads to a CaMK which we have called CaMK-VI. Over its entire length, CaMK-VI shares 99% amino acid sequence identity with the carboxy-terminus of predicted 750 amino-acid long human protein encoded by a cDNA clone called KIAA0369. Thus, it appears that the predicted KIAA0369 protein is an extended human homologue of CaMK-VI and this suggests that the gene encoding CARP/CaMK-VI may also generate a transcript encoding a rat extended CaMK-VI protein. Indeed, a rat DNA sequence encoding the amino-terminus of KIAA0369 has been deposited in Genbank (accession NR AA818566). In addition, the multiple CaMK-VI transcripts observed in our Northern blot analysis are well in line with this notion. Interestingly, the predicted 360 amino acid long doublecortin protein has a high degree of amino acid sequence identity (70%) with amino-terminal part of KIAA0369 but lacks catalytic kinase domain {des Portes et al., 1998; Gleeson et al., 1998} (see also Figure 2B). Thus, in human at least two related genes, doublecortin and KIAA0369, are present which encode proteins involved in kinase-mediated signal transduction. As both doublecortin and CARP are associated with epileptic seizures it shows a pivotal role of this gene family in epilepsy-related neuronal plasticity.

CARP mRNA is induced by kainate-induced seizures. In contrast, CaMK-VI isn't upregulated in these same animals.

This CARP-specific induction is thought to be the result of a negative feedback loop aimed at turning off kinase activity.

It is very well conceivable that calcium overload in a cell - triggered by kainate-induced seizures - leads to activation of different CaMKs and/or other kinases. This enhanced kinase activity induces CARP expression which subsequently acts as a modulator of this kinase activity, e.g. by acting as a substrate or by irreversible binding to activated kinases.

Thus the invention provides in one embodiment a novel mammalian calcium/calmodulin dependent protein kinase (CaMK-VI) and its encoding sequence, which kinase in the rat comprises the sequence 
MLELIEVNGTPGSQLSTPRSGKSPSPSPTSPGSLRKQRISQHGGSSTSLSSTKVCSSMDEN
DGPGEEESDEGFQIPATITERYKVGRTIGDGNFAVVKECIERSTAREYALKIIKKSKCRGK
EHMIQNEVSILRRVKHPNIVLLIEEMDVPTELYLVMELVKGGDLFDAITSTSKYTERDASG
MLYNLASAIKYLHSLNIVHRDIKPENLLVYEHQDGSKSLKLGDFGLATIVDGPLYTVCGTP
TYVAPEIIAETGYGLKVDIWAAGVITYILLCGFPPFRGSGDDQEVLFDQILMGQVDFPSPY
WDNVSDSAKELINMMLLVNVDQRFSAVQVLEHPWVNDDGLPENEHQLSVAGKIKKHFNTGP
KPSSTAAGVSVIAPDHGFTIKRSGSLDYYQQPGMYWIRPPLLIRRGRFSDEDATRM
 or a specific and/or active fragment or derivative thereof.

The sequence of the homologues of this rat kinase can of course be easily obtained by priming or other techniques in the art. For specific applications different parts of the sequences may be relevant and those parts alone are of course also part of the invention, as are derivatives of the original sequences, wherein several replacements of amino acids or the corresponding codons or basepairs can be made without changing the function of the kinase (at least without changing the kind of function, the rates of function can of course be affected (enhanced or deminished). Usually such changes can be made for changing e.g. the stability of a resulting encoded product. The product encoded by the nucleic acid or a degenerated variant thereof is of course also part of the invention. Thus in another embodiment the invention provides a mammalian protein kinase which is at least partially encoded by at least a specific part of the following DNA sequence;

CTCTGGCTCTTGGCTATTGTCAGGTCAAACACTAAGACTGTGTCCATGTTAGAACTCATAG
AAGTTAATGGAACCCCTGGCAGTCAGCTCTCCACTCCGCGCTCCGGCAAGTCACCAAGTCC
ATCGCCCACCAGCCCAGGAAGCCTGCGGAAGCAGAGGATCTCTCAACATGGCGGCTCCTCC
ACTTCACTTTCATCCACAAAAGTCTGCAGCTCAATGGATGAGAACGATGGCCCTGGGGAAG
AAGAGTCCGACGAAGGTTTCCAGATTCCTGCCACAATAACAGAGAGATACAAAGTCGGGAG
AACAATAGGAGATGGAAATTTCGCTGTTGTCAAGGAATGTATAGAGAGGTCGACTGCTCGG
GAGTATGCCCTGAAAATTATCAAGAAAAGTAAATGCCGAGGCAAAGAACACATGATCCAAA
ACGAGGTCTCCATCTTAAGGAGAGTGAAGCATCCGAACATTGTTCTCCTGATTGAAGAGAT
GGATGTGCCGACTGAACTATATCTTGTAATGGAGTTAGTAAAGGGTGGAGACCTTTTCGAT
GCCATCACTTCCACTAGCAAATACACAGAGCGGGATGCCAGTGGGATGCTGTACAACCTGG
CCAGTGCCATTAAATACCTGCACAGCCTGAACATCGTCCATCGTGACATCAAGCCAGAGAA

CCTGCTGGTGTATGAGCACCAGGATGGCAGTAAGTCACTCAAGTTGGGTGACTTTGGCCTG
GCCACAATTGTCGATGGCCCCCTGTACACAGTCTGTGGCACCCCAACTTATGTGGCTCCAG
AAATCATTGCAGAGACTGGATATGGCCTCAAGGTGGACATCTGGGCAGCTGGCGTGATCAC
TTATATCCTGTTGTGTGGTTTCCCTCCATTCCGTGGAAGTGGGGATGACCAGGAGGTGCTT
TTTGACCAGATCTTGATGGGCCAAGTGGACTTTCCATCTCCGTATTGGGACAATGTGTCAG
ATTCTGCTAAGGAGCTCATCAACATGATGCTGTTGGTTAACGTGGACCAGAGATTTTCAGC
CGTGCAAGTCCTTGAGCACCCCTGGGTTAACGATGATGGCCTCCCAGAAAATGAACATCAG
CTGTCAGTAGCTGGCAAGATCAAGAAGCATTTCAACACGGGCCCCAAGCCGAGCAGCACGG
CAGCTGGAGTTTCTGTAATAGCACCGGACCACGGGTTTACCATCAAGAGATCAGGGTCTTT
GGACTACTACCAACAACCAGGAATGTATTGGATAAGACCACCGCTCTTGATAAGGAGAGGC
AGGTTTTCCGACGAAGACGCAACCAGGATGTGAGGGGCCGGTACAAGGCACAGCCAGCTCC
ACCAGAACTCAACTCGGAATCAGAAGACTACTCCCCCAGCTCCTCTGAGACTGTCCGCTCC
CCCAATTCGCCCTTTTAATAAGACCCCTTCACTCGAAGTCCTAGCTTAACCCTTTAAGACT
CTGAGA[?]TCCCCCTCCCCCCAAACTTCTGTAAAACAGGTTCATCTGATCTACCTAGCACTC
AGTGCTGAACGGCAGAACAGAAAGTATATTTCGGGTACCTTTGTAGCAAGTTCCCTTTACT
GAATGAGAAGGCATGTGGTGTTTGTGACAACGGTAATTTGCTGCTAATAGGGGTCCTTAAA
GGGTTGAGGCTAATTTGCGATTTTTTTTCTTTTAACATAGAAGTAACGAATGTTTTCATCA
GTCAGACTAGGATCTGCAGTATGTAACATAGCACATGTTAGCCCTCTGAGTGCATAGAATT
TGCTTGAGAATCCTTGCTGGGAATTTTTCAGACCCTTGAGTGTATTCACACATATTTCTT
GATTTTACTGCAGACCAAGGGGCGTCATTGAGACCCAAAATGTCCACCCAAGAAGAGTATT
GGGGAATGACACATTGCCAATTCTTCTCAGTGGGCTTAGACAATGACGGGTTCTACAGCAT
CAGCAGATGCACCCCTCCTTCTTAGCCATCTTATGAAGAAGGGAAGTCCCTTTCCCTACAT
AGTAAGCTGCAGAGCCTTCTCTGCTCTGATTGGAGGGAGTTAAGGTTTGTGGCTGCCTATC
CCAAGTGATCGACAGTGTCTCTCC

Of this nucleic acid derivatives and/or fragments as meant hereinbefore for proteins are of course also included. A derivative and/or fragment will have the same or similar function and/or structure (in kind not necessarily in amount) in the resulting product. As disclosed herein the gene encoding the novel kinase has an alternative splice product involved in feedback. This is of course also an embodiment of the present invention, both on the nucleic acid level and the protein level, which can be applied in feedback of any kinase related to the presently invented kinase. Thus in another embodiment the present invention provides a mammalian calcium/calmodulin dependent protein kinase related peptide (CARP) which in the rat has the amino acid sequence
MLELIEVNGTPGSQLSTPRSGKSPSPSPTSPGSLRKQRDLYRPLSSDDLDSGDSV
and in the mouse
MLELIEVNGTPGSQLSTPRSGKSPSPSPTSPGSLRKQRDLYRPLSSDDLDSVGDSV
or a derivative or a specific and/or active fragment thereof.

Here derivatives and/or fragments are intended in the same manner as hereinbefore. Homologues in other species or alternative splicing products of reated kinases can now easily be produced according to the invention. As stated the product of this alternative splice product or its copy is also provided.

Thus the invention provides a mammalian calcium/calmodulin dependent protein kinase related peptide (CARP) which is at least partially encoded by at least a specific part of the following DNA sequence

GGCTATTGTCAGGTCAAACACTAAGACTGTGTCCATGTTAGAACTCATAGAAGTTAATGGA
ACCCCTGGCAGTCAGCTCTCCACTCCGCTCCGGCAAGTCACCAAGTCCATCGCCCACCA
GCCCAGGAAGCCTGCGGAAGCAGAGGACCTGTACCGCCCTCTCTCGTCGGATGATTTGGA
CTCAGGAGACTCAGTGTAAAAGACACAGTGGAGCGATGTGTATGGTTTGTGATGGGATCAA
GGTTTCCCATTTCCTAAGCAAGCATCTAACAGCAAAATACACAAAAGAGTTTTGCATAAGC
AATTAAATTATACAAAAGCAAGAGTGCAGACATTTTCTACTCGGCGGTATTCTTTGAAAGA
AAAAAAAAACTTTTTAAAGCATCTCCTCCATTTGATGTAACCGAGGAGAGTTTGAATACA
ATATTTGTTCATCACTGAGGATTTTATGACTAAGGTCTGGTTCCAGATAAATTTCATATAT
GACTCCAATATTAAGTACAAGAATCACCCTAACGATCCAGCATTATGGAGTCACCATTTCT
ATAAATGAGCCTGCTTTTTAATCAGTAAGTAGATATATAAATACAATTTTTCTGTACTTAG
TATCTTTTACCTTTTTAGTTTAAATTTTTTAAAAAAATCAGACAGCTAATTAGTTATTTCA
TTGCAAAATAACATAAAAGAATCTCTTCAGGATTTTTTATTTGAGTGAATACGTCTGAAGG
GGAGTGGAGGAACTCCGTAAGAGACGTCTCCCAGAGACCAACATTAGGCAGCGAGAACGTT
TCTGGTTTTTTTTTTTTTTTTTTTTGTTTTTGTTTTTTGCCACATGGATTTTCTTTTCCCA
GATTTTTATAAAAATAGTTTCAACTACATGGAAACATGAATAACTTGGCATTATTATTAAT
TTTATAATGGTTCCCAACAGCAACAACAGTATATATATCTTTTTCAAGTGTGTAAAGTTTT
TTGACCTAAAACAAATGGGACTTGTACTGTTTTTCAAAAAATAAACTCAGTTTGAGAATTG
GAATTCTTCTACACAGAGCTTGTACGTTGGGCCTCCAGGTAGCTAAACTCCATTCTGATCA
TCCTGCATCCCGTTGTTTGGGATATCTTCACATGCAAAACTCCTTTTGTGTCAAACCGAGT
GTGTCTATGATTATAACCCACTGTTAAAATTAACCTACTAATTCTTCTCCTTTAGTCCTCT
ACTTTCAAAAGCGTGAGTCAGCTTTGCTTAAATGTAAAACAGTTATTTTTTAAATCTAAAT

GTTAGAAATCAATGTGTACGGTGTATTGTATTTAGACCATACCCGTGACCGTTTCTTTTTA
CTAGCTCAAACACTAACAATTTAAGACTTACAAAGACATGGGTTTTATATTCGCCAGGCTT
GACATATCTCAGTAGGGTGACAGATTTAAAATGTATGGTGCCATTTGGAGCCTTTTACTTG
AGATTTCTTTCAAATTTCACTTGAAAACATTTTGAAAATGCCTGATTCAAGATGGAGAGGA
GTCGCCTTGTCATACGGTCTCAAATGTTATTGATTTATTTCTCTCAGTAGACTGTTCGACT
AGCAATTTTTCCCATCTGGTTTATCGTTACCTGTGCTAGCATCTCACTTGACAAGTGAATA
AAATATTCCAACTGAAAAAAGAAAAATAAGACCTCCAGGCAACCTACCAGCATTTGAAAAG
TTAGCCGAGTGATAATTAGTGCCTCATAACCCTGCCTGTTAGAGAGGAGAGGGCACTTCTT
TGGCGTGTTATTTGAGAACTGCAGACTGATCCCTCCATCTTGAAAGCACAGAGAATGATCA
TACCTGTTCCAGACTTCGGATTTTAAAACTGTTTCCAATAGACACGAACAGAGACTCCTGC
TGAGATCAAAGGGCCGCTTTGTCTTTAGCTGAAACAGTAAACATCTGGGAGGATGCTGTGC
TGCCTGTTGGAGTGTTACGTCTTTGTAAGATGCATTGCTTAATATAGTCCAACCCGTAAAA
GATTAGTACAGATTTGACTAACCTGTCAAAAAATGCAATGAAAGCAGTGGGTCAGATGGTC
CAGAACAAAACATAAATTCCAGTAATTTCAACTCCCCTATGAAGTCAATCTTATGGACGAC
TACTGCTAGGAGAAACCTAATAAATGTTATCTGCTATAAAAATCGTGGCTTTGTTTAGACA
CAGCATAGAGTAGACTTGGACTAATTGCCAGTAGGTATTGTGGCCAACTTTCCATTCAAGC
AGTTCCAGAGAGATGCAATCCCATGGCATTTCATCCTGGAACATACGATTTTTATTAAAGG
ATTGTTCAAGGGCCATATATCTCATTTACTTTTGAAACTAAAGTCCCTCTGAGATTGAAAC
AACACACCCTGAGATCTCTTTCTATGCACGGAATAATTCTATTATCCAAAAGCAGAGTTTT
TGAACATCCAAGCAGCCCACCAGGTCATTAGTAAAGACCCAATGGGCAATTAGCAGTCACT
CAAGACGTGGCTGCTTTTAGAATCCTTCTAACATCATTAGTCGCTGAACTCTAAAAATGAC
CTTCTGAGGAAAGAGCTCCAGCCAACAAAAGCCACATGCTTTTCTCTCACTGATAAAGCCT
CTGCTGTGTTGACCTTTCAGGCCTTTGCATGTATGGTTACATTTTTTTAGACATACCTAGA
AAGAAAGATGCTATAAAGAGTCCCCTGCTTTCAGCATTTTATGAGCTAAAACCCTAGTGAT
GCACAAAATGTGTTGCTCTGCGTGAACTCCGAAGCCATGTGGGAAAGTCATTGTCATTTCA
GCAGAAGGTCTGTACTCTTGTAGGTTTCTAAACCGGAAGTTTGTGCTCCACCTATATGTAC
GCATGCCTGCAATGCACACATGAGGTCACCTTAACATGTCAAGATCTGATCCCGTTGTCTG
CCAACCCCAGAGCAGCTCTGACACCTATGCTCCCCTGCCAAGCAGCGAGGGCACTCGCCCT
CCCCTTCCTTGCAGCCGCACCGAGCTGTACTTTTGTTTCCATTTAAAAGAACTGAATTCCA
AAAGCCTCGTTTTAAAAAAGCAATTGGGAATGAAAATACATAAGAATAATCCTAAAAGATA
AATCCATATATTTAACCTGTTGAACTACTAGGTTTTTTTTTCTTTTCCTGGTGGCCATAT
ATCTATACAGGTGTGTAGGTGTGCCTA
CATGAATATCTGTGTTTGTATATAGAACATGAAGAATAAAGGATGATATTTGTGATATGGT
CTTATAAGTTACTGGCTCCTGTAGAATTAACTGTGAAGCTTTATGTGATAAAGTTGTGACG
TATGTATTAGTTGGCACATGTTTAAGCTCAAAACAAGTTGGCTAAATTATCATGAAGCAGT
ACCAACTGTTCACGGGTACGAATTTATCCAAGACCGATTGGAATCCAGACAAACAAAAAAA

TTGAAGTCCAAGCAAATCCAATCTTACAGTGATTGATAAATGTAAATACTCATTGATGTTT
TCACTGTAAATTCTTGATGCTCAGCCAGTAATCTGACAGAATGCAGTGACACTTTCCTGTT
GTGTGTGTACTGATGACTACAGCGATTATCAATGGGAAATTCACCAAACAAACTGATTTCT
AGACTTGTGGACAGTTGAGTGTTTGTGCTGAAATAAAAGTAGAAAAGGTCAAAGTAAATTC
GGTTTGAAGCCTTGTTATGAAGCCAGACAACATGTAAGGCCATGCTGTGTGTCCTTTTTAT
AAACTGTTTTGGAGACACTTGCTGTGTGTGCATGTGTGTCTACATGAGTGTATACACAC
TTGAAAATGCTTATTGACTCAAGTTCACTGTCTGTATACATCTGTGCCTATCTGCATTGGT
CTGGCCAGCGCCTCTAACTGTGCATGGTAGTGACAGCCCTGTGTGATTGGATGAACCCACA
GAAAAATAGTGTGGAAATCAGAGCTGCGTCAAACTCATTATAACAATTGCCTGATATAGCA
TATTAAATTGTCAGCTGAAAAACGTTTGTAATGTTGTGCCTGTAACATGTGTAGGGAAAAC
TGGAAAATCTGAGGCCCTCACTATAATTTTAAGGGTGCATGACATGTGGTGTCTTTAAAGA
TGTATCTTATTGTAATAAAAGGTGATGGATAAATTCTGAGGTAGCGTAAGTGAAGCATGAA
ACGCTAGGTGTTTGTATTATATATGGCCACACAATATTTTTGAAGGCAGGGTAGTAAATGT
TTGAAGGTTAATAGGCAAAGAATATGGAAGTTAATTTTACTGTGATGTGTTTTTGAGGTGT
TTTACACATTGCCAGACAAGGAACTTAGTTCCTGTTTCCCCCCTGGGACTCTTTGAAACAC
TATGAATGTTACAAAGTTGGATTTTCTTTATAAAGGTCGCAAACCATTTGCAGTTGGAGTT
GCTTGGATGTATAGTCTCCTCTTTGGGGAAAAAAACCACTTTGGTATTAAAATGGTTTAAG
CACTAATACATTGGGAAATACGTCGTCTGAGGCCATCTGCAGACATCTGTAGAGATGAGAA
CTAATGAAATGTGTTACTGAACTACCCAGTTTTGATAGCTTCCATGCCTCTCTGCTGTAGT
CTCCTCGAAAATGCTGTACCTTGTGTTTCACATGCAACCATGTTACTCTCTCCCGAACTTA
CCTTTGTGACACTTAGCAGCTTAGCCAAACCACGTAATAAACAGGAAGAACTGCCAAAAAA
AAAAAAAAAAAAAAAAAAA

As stated the invention also provides a recombinant and/or isolated nucleic acid molecule encoding at least a specific and/or active part of a mammalian calcium/calmodulin dependent protein kinase (CaMK-VI)and/or a recombinant and/or isolated nucleic acid molecule encoding at least a specific and/or active part of a mammalian calcium/calmodulin dependent protein kinase related peptide (CARP).

Also part of the invention is a cloning and/or expression vector comprising an isolated and/or recombinant nucleic acid molecule as provided above.

Also part of the invention is a mammalian host cell comprising a recombinant and/or isolated nucleic acid or a vector as represented above. Typical applications of the molecules or their precursors according to the invention are the treatment and/or prevention of seizures and/or damage to the central nervous system, in particular the hippocampus. In general the invention provides a method for down regulating calcium/calmodulin dependent protein kinase activity in cells comprising providing said cells with a mammalian calcium/calmodulin dependent protein kinase related peptide (CARP) according to the invention.

Our invention is relevant for a number of diseases. Acute brain insults as stroke, trauma, hypoxia, ischemia, but also some neurodegenerative diseases such as Alzheimer are associated with high local concentrations of excitatory amino acids, in particular glutamate. These glutamate concentrations will lead to excessive Ca2+ influx and consequent activation of numerous calcium-dependent kinases which will ultimately lead to cell death and thus neurodegeneration. As a consequence, glutamate is released by these dying cells which, in their turn, will threaten neighbouring cells by introducing excessive calcium in the cytoplasm (for review see Pettmann and Henderson, 1998). As CARP is capable of down-regulating calcium-dependent kinases, this self-propagating cell-destruction programme, associated with the diseases mentioned above, is likely to be blocked by CARP and thus, excessive neurodegenerative processes can be prevented by CARP treatment.

In addition, the induction of proliferation of T-lymphocytes has been associated with activated CaMK-IV (Gringhuis et al, 1997; Marklund et al, 1994) which, of course, can be also be blocked by CARP. Thus, certain forms of leukemia, which are characterized by aberrant T-lymphocyte proliferation may be blocked by CARP.

The action of CARP is tested in the following models:
1. Different cell lines, i.e. the neuronal cell lines PC12, HT22, H19 cell as well as several neuroblastoma cells and several non-neuronal cell lines such as Jurkatt cells, derived from lymphocytes will be challenged with apoptosis-inducing agents. The rate of apoptosis is monitored by biochemical means such as DNA laddering, histological means i.e. TUNEL staining, FACS scanning etc. and compared with the reduced rate of apoptosis in which CARP is overexpressed by means of transfecting cells with expression plasmids pcDNA 3.1 containing CARP in a sense orientation.
2. Synthetic full length CARP peptide is used to manipulate the activity of purified MAP kinases, CaM-kinases and other calcium-dependent kinases in an *in vitro* kinase assay (see e.g. Veeranna et al, 1998).

Treatment of patients with with acute brain insults mentioned above, will occur by local i.c.v. administration of the CARP peptide, by local expression of CARP via viral infection of recombinant adenoviruses containing the CARP construct, or by development of agents which induce endogeneous CARP expression. Leukemia can be treated by intravenous administration of the CARP peptides or modifications thereof.

The invention will be explained in more detail in the following detailed description.

### Detailed description.

### Materials and methods

### Animal treatments

Young adult male Wistar rats (180-200 g) from Charles River were housed under standard light conditions; 12 hr. light/dark cycle with free access to food and water. Eight mg per kg body weight kainic acid was administered intraperitonially. Control treatments consisted of administration of vehicle solutions only. Three hours after injection, the rats were decapitated, the brains were dissected and immediately frozen and stored at -80°C until use. All animal experiments were conducted according to the European Communities Council Directive 86/609/EEC. The protocols were approved by the animal Care Committee of the Faculty of Medicine, University of Leiden, The Netherlands (UDEC no 9569).

### Isolation off full-length CARP cDNA clones and a partial CaMK-VI cDNA clone.

A CARP cDNA fragment, generated by differential display (DD) and induced by adrenalectomy and kainic acid administration {Vreugdenhil et al., 1996} was excised from the nitro-cellulose membrane, eluted and subcloned in pGEM-T (Promega) according to Engels and Vreugdenhil (manuscript in preparation). DNA sequence analysis was performed using a ABI 377 automated sequencer and a Dye terminator cycle sequence kit (Perkin Elmer) according to the instructions of the manufacturer. Based on the DNA sequence obtained, synthetic oligonucleotides were synthesised (Perkin Elmer, Nieuwerkerk a/d IJssel, the Netherlands) and used in the marathon cDNA amplification kit (Clontech), which is a modification of a polymerase chain reaction (PCR)-based strategy called Rapid Amplification of cDNA Ends {Frohman et al., 1988}. PCR-fragments were size-separated on 1.2 % agarose gels, blotted and hybridised with ³²P-labelled oligonucleotides, designed from internal DNA sequence of the DD fragment. Positive PCR-fragments were excised from the agarose gel and analysed directly by DNA sequence analysis or first subcloned in pGEM-T (Promega). DNA sequence analysis was performed by a "DNA walking" technique towards the 5' end and 3' end of the transcript using synthetic oligonucleotides derived from novel DNA sequence information. This strategy resulted in a final stretch of 4746 nucleotides. The identity was verified by the complete amplification of this sequence using extreme 5' and 3' synthetic oligonuceotides and by Northern blot analysis. This sequence has been deposited to Genbank and is available under accession NR AF045469.

The CaMK-VI cDNA clone with a length of 2031 nucleotides was isolated and characterised by PCR using an oligonucleotide 5'-CTCTGGCTCTTGGCTATTGTCAGG-3' derived from the 5'end of the CARP cDNA clone and an oligonucleotide 5'-GGAGAGACACTGTCGATCACTTGG-3' corresponding to position 2092 to 2115 of Genbank entry accession NR U78857. The resulting cDNA fragment was cloned in pGEM-T and analysed by DNA sequencing.

### Northern blot analysis

Total RNA was isolated from rat hippocampi using TRIzol™ (Gibco-BRL) according to the manufacturer's instructions.

Total RNA (approximately 20 µg per lane) was size-separated on a 1% formamide gels and blotted onto Hybond-N filters according to standard methods {Sambrook et al., 1989}.

Filters were hybridised O/N at 65°C in buffer containing 0.4 M Na2HPO4, 0.1 M NaH2PO4, 7% SDS and 1mM EDTA. Filters were washed at 68°C, twice in 2xSSC/ 0.5%SDS, and once in 0.2xSSC/0.5%SDS. Each wash step was performed for 10 min.

Filters were exposed to Kodak X-Omat AR films for one week.

To correct for differences in RNA levels and RNA quality, filters were reprobed with a glyceraldehyde-3 phosphate dehydrogenase (GAPDH) probe. Hybridisation, washing and exposure was performed as described above.

DNA probes were labelled with ³²P α-dCTP using the multiprime DNA labelling system kit (Amersham, England) according to the manufacturer's instructions. A CARP probe was prepared by RT-PCR amplification of a part of the 3'-untranslated tail (bp 880 to 4657) with synthetic oligonucleotides 5'-CATGGAAACATGAATAAC-3' and 5'-CGGGAGAGAGTAACATGG-3' using hippocampal cDNA derived from a kainic acid-treated rat as a template. A CaMK-VI probe was prepared by PCR amplification of the middle part of the coding region encompassing bp 769 to 2040 by using synthetic oligonucleotides 5'-AGTCTGTGGCACCCCAACTT-3' and 5'-GGAGAGACACTGTCGATCACTTGG-3' and using the cloned CaMK-VI cDNA (see above) as a template. A GAPDH probe was prepared by PCR amplification of residue 149 to 515 corresponding to EMBL database entry NR X02231 by using synthetic oligonucleotides 5'-GACATTGTTGCCATCAACGACC-3' and 5'-TGCATTGCTGACAATCTTGAGG-3', using hippocampal cDNA described above as a template.

All resulting DNA fragments were gel-purified and their identity was verified by DNA sequence analysis of both ends.

Hybridisation signals were quantified using an Olympus image analysis system (Paes, The Netherlands), equipped with a Cue CCD camera. The film background was subtracted after shading correction.

### In situ hybridisation

*In situ* hybridisation experiments were performed according {Meijer and de Kloet, 1994}. Briefly, serial coronal sections (20 µm) were cut through different brain regions. The sections were thaw mounted on poly L-lysine coated slides and stored at -80°C. Hybridisation was performed using the CARP-specific 180 bp cDNA fragment, identified by DD. This fragment corresponds to bp 3268 to 3443 and doesn't overlap with CaMK-VI. This fragment was polished using PfuI DNA polymerase (Stratagene) and ligated in the SrfI site of the vector pScript (Stratagene). Both the sense and the antisense cRNA probes were generated using ³⁵S labelled UTP and T7 or T3 polymerase. For hybridisation, 100 ml of a probe with a concentration of 2,5x10⁷ cpm/ml was used. The sections were exposed for seven days to a Kodak X-OMAT AR film.

### Results

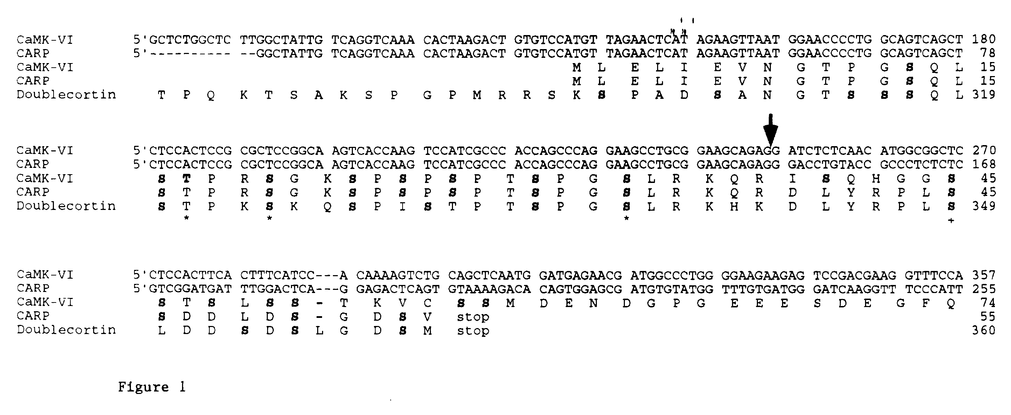

We have used the differential display (DD) technique to identify kainate-responsive genes which are under the control of corticosteroids {Vreugdenhil et al., 1996a; Vreugdenhil et al., 1996b}. Analysis of kainate-upregulated genes by *in situ* hybridisation indicated that one product is specifically induced in subregions of the hippocampus (see also below) during kainate-induced seizures but not by administration of sub-treshhold doses of kainate (data not shown). We have cloned the corresponding cDNA encompassing 4746 bp with a stretch of 41 adenosine's at the 3' end and a classical poly-adenylation signal AATAAA present 20 bp upstream of the polyA tail. Northern blot analysis of hippocampal total RNA derived from a kainic acid-treated rat (see later) resulted in the identification of a 4950 bases-long transcript indicating the cloning of a nearly full-length cDNA. Open reading frame (ORF) analysis demonstrated the presence of many small potential peptide/protein sequences. To determine the most likely candidate we have cloned the homologous cDNA in mouse (data not shown) and compared the potential ORFs of mouse and rat. The only conserved ORF is located at the extreme 5'-end and encodes a peptide of 55 amino acids with a calculated pI of 4.69 (see Figure 1A). A remarkable feature of the peptide is the presence of 12 serine residues and 7 proline residues. No apparent consensus signal peptide or transmembrane regions were identified. Post-translational motifs were searched for by PROSITE {Bairoch, 1993} and suggested phosphorylation sites at amino acid T17, S20 and S33 by protein kinase C and S45 by casein kinase (see Figure 1), showing functional significance of the peptide in signal transduction pathways.

Searches of both protein and DNA databases did not reveal significant homology of the 3' 4600 bp with any known sequence. In complete contrast, the remaining 5' end shared 100% sequence identity with candidate plasticity gene 16 (cpg16; Genbank accession NR U78857). As yet, no literature reporting the structure encoded by cpg16 is available. To verify the sequence of cpg16 and to study its possible relationship with our cDNA we have cloned by PCR a 2031 bp cDNA clone, called CaMK-VI (see below) by using cpg16 specific oligonucleotides. DNA sequence analysis confirmed the cpg16 sequence except that an additional 74 nucleotides are inserted in CaMK-VI at position 1273 of the original cpg16 clone. This causes a frame shift and an in frame stopcodon 130 nucleotides downstream of the point of insertion and thus results in a complete different carboxy terminus than predicted in the cpg16 cDNA clone. The putative human homologue of cpg16, KIAA0369 ({Nagase et al., 1997} (Genbank accession NR #AB002367) predicts a protein which is identical to the carboxy-terminus of by the CaMK-VI clone.

Therefore, we conclude that our CaMK-VI clone is authentic and represents an expressed transcript species in the hippocampus.

CaMK-VI contains an ORF of 1299 bp encoding a 422 amino-acid long protein which contains all of the amino acids and motifs which are characteristic for the superfamily of CaM kinases {234}. The 267 amino acids encompassing the catalytic domain exhibit 40-45% sequence identity with the catalytic domains of other CaMKs while the regulatory domains are less conserved (10-35% amino acid sequence identity). These figures are well in line with those reported for other members of the CaMK family {Nairn and Picciotto, 1994; Hanson and Schulman, 1992; Haribabu et al., 1995}. In particular, the structural organisation is reminiscent of the CaMK-IV protein in that a catalytic kinase domain is preceded by an approximately 60 amino acid long serine-rich N-terminal extension. Though the overall sequence identity is low (18%), 17 serine residues are present in CaMK-VI, five of which can be aligned with serine residues of CaMK-IV. It is in this N-terminal extension that the structure of serine-rich CARP overlaps with CaMK-VI. We have concluded that CaMK-VI is a novel member of the CaMK family. Therefore, we have called this clone CaMK-VI.

Alignment of the CaMK-VI cDNA clone with the cDNA clone identified by DD gives identical sequences of the first 147 bp while no homologous regions were found in the remaining parts (see figure 1). The sequences diverge at a DNA motif resembling exon-exon boundaries (AGGA and AGGG) suggesting that our cDNA clone and CaMK-VI are derived by alternative splicing of the same gene. At the protein level, the amino-terminal 38 amino acids are identical; of the remaining 17 amino acids only 4 are identical, 3 of which are serine residues. We propose to term the 55 amino acid peptide encoded by our cDNA clone CaMKVI-related peptide (CARP).

In addition to CaMK-VI, CARP has 70% sequence identity with human doublecortin, a brain specific gene which has recently been implicated in X-linked lissencephaly and the doublecortex syndrome and which is thought to function as a signalling protein {des Portes et al., 1998; Gleeson et al., 1998}. Two of the four kinase phosphorylation sites, i.e. T17 and S30 are conserved. Interestingly, the carboxy-terminal 17 amino acids of CARP which are not homologous to CaMK-VI shares high amino acid sequence identity (82%) with doublecortin. In addition, the length of the carboxy terminal corresponds precisely with that of doublecortin suggesting a common evolutionary origin of the last coding exon for CARP and doublecortin.

### Expression of CARP and CaMK-VI in the rat hippocampus.

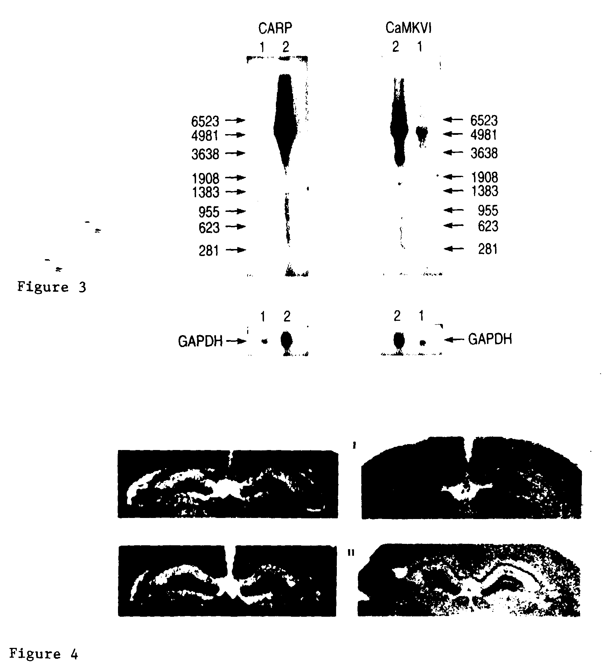

The identical 5'-ends of the CARP and CaMK-VI cDNAs suggests a similar transcription regulation of both transcripts during kainate-induced seizures. To study this and to identify the length and number of CARP and CaMK-VI transcripts, Northern blot analysis was performed using total RNA derived from hippocampi of vehicle-treated animals and animals showing kainate-induced seizures. Nearly no CARP transcript was detectable in vehicle-treated animals (see figure 3, CARP, lane 1). However, a dramatic 30 fold induction of a CARP RNA species ( 4.9 kb; see figure 3, CARP, lane 2) was found in animals with kainate-induced seizures. In addition, two minor transcripts of approximately 8.3 kb and 4.0 kb were observed. Surprisingly, however, the expression of CaMK-VI differed from that of CARP in that no clear upregulation was seen after kainic acid treatment. Six different CaMK-VI transcripts, one major species with a length of 5.0 kb and five minor ( 8.3 kb, 7.0 kb, 4.0 kb, 3.4 kb and 1.4 kb) were detected in both vehicle-treated and kainic acid-treated animals (see figure 3, CaMK-VI, lane 1 and 2, see also table I). Ethidium bromide staining of the gel indicated differences in the amount of RNA loaded in the different lanes (data not shown). This was confirmed by hybridisation with the GAPDH probe (see figure 3, bottom panels). After correction for differences in the amount of RNA, no clear difference was observed in the expression levels of five of the six CaMK-VI transcripts after kainic acid-treatment (see table I). However, one CaMK-VI transcript with a length of 3.4 kb was upregulated after kainate-induced seizures by a factor 2.5 (see table I). In conclusion, these experiments show that at least six different CaMK-VI transcripts are present in hippocampus, of which one is differentially regulated by kainic-acid treatment. In contrast, CARP transcript levels are very low in hippocampus under normal conditions, while kainic-acid treatment gives rise to a dramatic induction of in particular a 4.9 kb CARP transcript.

To study the cellular localisation of CARP and CaMK-VI mRNAs and to confirm our Northern blot analysis we have used in situ hybridisation. In accordance with our Northern blot analysis, CARP mRNA was not detected in the brain of vehicle treated animals (see figure 4). Kainate-induced seizures, however, led to induction of CARP mRNA in the hippocampus within 3 hours, but not in any other part of the brain (see Figure 4). CARP mRNA was prominently present in the dentate gyrus and to a lesser extent in the pyramidal layers CA1 and CA2. CARP expression was not detected in CA3. This CARP mRNA induction is in line with our DD results {Vreugdenhil et al., 1996a} and occurs in discrete regions of the rat hippocampus after kainate-induced seizures. In accordance with our Northern blot analysis CaMK-VI is expressed at similar levels in both vehicle-treated animals and animals with kainate-induced seizures. Highest expression was found in the dentate gyrus while the signal was less in CA1 and CA2. In contrast to CARP, CaMK-VI mRNA was also detected in the CA3 region.

In conclusion, our Northern blot analysis and in situ hybridisation experiments showed differential regulation of CARP and CaMK-VI transcripts by kainate-induced seizures.

CARP mRNA is normally below detection levels but is dramatically induced by seizure activity in the hippocampus, in particular in the dentate gyrus. CaMK-VI expression is already found under steady state conditions and this expression is not affected by seizure activity.

Our *in situ* hybridisation experiments confirm our Northern blot analysis and show that CARP is expressed only in the dentate gyrus and pyramidal neurones of CA1 and CA2. This cell-specific expression of CARP is reminiscent of the restricted expression pattern of the transcripts generated by the CaMK-IV gene. In contrast to the wide distribution of other CaMK mRNA species, the expression of the CaMK-IV gene is highly restricted to specific neuronal cell populations {Sakagami and Kondo, 1993; Sakagami and Kondo, 1996} T-lymphocytes {Hanissian et al., 1993; Marklund et al., 1994; Gringhuis et al., 1997} and germ cells {Sun and Means, 1995, Sun et al., 1995}. This suggests that CaMK-IV and VI may form a distinct group within the superfamily of CaMKs characterised by a restricted expression, serine rich N-termini involved in auto-inhibition and alternative splicing of their corresponding genes resulting in completely different proteins/peptides.

In conclusion, we have cloned and identified a cDNA clone, called CARP which is specifically upregulated by kainate-induced seizures and which is likely generated by alternative splicing of a putative CaMK gene. Expression of CARP may result in a peptide which modulate kinase activity. Our findings further substantiate a important role for kinases in the course of kainate-induced seizures. We postulate a model in which calcium overload leads to overactivation of kinases.

This triggers CARP expression which, through a negative feedback action, restores kinase activity. The cloning of CARP and related cDNAs opens up the possibility to test this hypothesis.

### Figure legend.

*Figure 1*.
   Alignment of the 5' end of CARP and CaMK-VI cDNA sequences and their predicted amino acid sequences and of the carboxy-terminus of the predicted human doublecortin amino acid sequence. The potential exon-exon boundary is indicated by an closed arrow. Serine residues are shown in bold. Putative phosphorylation sites are shown by asterisks.
*Figure 2*.
   Structure and alignment the predicted rat CaMK-VI, CaMK-IV CARP and human doublecortin and KIAA0369 proteins. A: Alignment of the predicted primary structure of CaMK-VI with rat β-CaMK-IV. The putative catalytic domain is indicated with a solid line above the sequences; the regulatory domain is boxed. Lys 99 of CaMK-IV and Lys 112 of CaMK-VI involved in ATP binding, and Thr 244 of CaMK-IV and the corresponding Thr 239 of CaMK-VI, which are thought to be crucial for activation of the kinases by phosphorylation are shown in bold. The GTPXXXAPE motif characterising Ser/Thr kinases is found at position 227 to 234 in CaMK-IV and position 242 to 250 in CaMK-VI and is shown in italics. B: Schematic representation and alignment of CARP, CaMK-VI, CaMK-IV, doublecortin and KIAA0369. The different functional domains are boxed and indicated below the alignment. Percentage of amino acid sequence identity is indicated between the alignments. For further details: see text.
*Figure 3*.
   Expression of CARP and CaMK-VI in the rat hippocampus as determined by Northern blot analysis. Total RNA was isolated from the hippocampus of a vehicle-treated rat (lane 1) and kainic acid treated rat (lane 2). The blots were probed with ³²P-labelled CARP (left panel) and CaMK-VI (right panel) labelled DNA probes and exposed to Kodak X-OMAT AR films. To correct for differences in RNA quantity and quality, blots were reprobed with a GAPDH cDNA probe (shown at the bottom of both panels). The position of RNA markers and their molecular weights in bases are indicated by arrows.
Figure 4.
   *In situ* hybridisation analysis of CARP and CaMK-VI in the rat hippocampus. panel I shows a section of a vehicle-treated animal and panel II shows a brain section derived from an animal with kainate-induced seizures. An asterisk in panel II indicates the CA3 region lacking CARP expression. Further are indicated: the pyramidal layers CA1 and CA2, and the dentate gyrus (DG). Control experiments using radiolabelled sense-RNA probe didn't give any signal above background (data not shown).

### Table I

The effect of kainic acid administration on CARP and CaMK-VI mRNA levels.

Hybridisation signals were quantified using an Olympus image analysis system equipped with a Cue CCD camera. CARP and CaMK-VI signals were corrected by signals obtained from the GAPDH probe. Numbers given in the table represent the signals obtained after kainic acid administration divided by the signals obtained after vehicle treatment. As no signal for the CARP transcripts of 8.3 kb and 4.0 kb was detected in the vehicle-treated animals their induction was not determined (nd).

### REFERENCES

Bairoch A (1993) The PROSITE dictionary of sites and patterns in proteins, its current status. Nucleic Acids Res 21:3097-3103.
Buckmaster PS, Dudek FE (1997) Neuron loss, granule cell axon reorganization, and functional changes in the dentate gyrus of epileptic kainate-treated rats. J Comp Neurol 385:385-404.
Chatila T, Anderson KA, Ho N, Means AR (1996) A unique phosphorylation-dependent mechanism for the activation of Ca2+/calmodulin-dependent protein kinase type IV/GR. J Biol Chem 271:21542-21548.
des Portes V, Pinard JM, Billuart P, Vinet MC, Koulakoff A, Carrie A, Gelot A, Dupuis E, Motte J, Berwald-Netter Y, Catala M, Kahn A, Beldjord C, Chelly J (1998) A novel CNS gene required for neuronal migration and involved in X-linked subcortical laminar heterotopia and lissencephaly syndrome. Cell 92:51-61.
Dobyns WB, Truwit CL (1995) Lissencephaly and other malformations of cortical development: 1995 update. Neuropediatrics 26:132-147.
Frohman MA, Dush MK, Martin GR (1988) Rapid production of full-length cDNAs from rare transcripts: amplification using a single gene-specific oligonucleotide primer. Proc Natl Acad Sci U S A 85:8998-9002.
Gass P, Herdegen T, Bravo R, Kiessling M (1994) High induction threshold for transcription factor KROX-20 in the rat brain: partial co-expression with heat shock protein 70 following limbic seizures. Brain Res Mol Brain Res 23:292-298.
Gass P, Bruehl C, Herdegen T, Kiessling M, Lutzenburg M, Witte OW (1997) Induction of FOS and JUN proteins during focal epilepsy: congruences with and differences to [14C]deoxyglucose metabolism. Brain Res Mol Brain Res 46:177-184.
Gleeson JG, Allen KM, Fox JW, Lamperti ED, Berkovic S, Scheffer I, Cooper EC, Dobyns WB, Minnerath SR, Ross ME, Walsh CA (1998) Doublecortin, a brain-specific gene mutated in human X-linked lissencephaly and double cortex syndrome, encodes a putative signaling protein. Cell 92:63-72.
Gringhuis SI, de Leij LF, Wayman GA, Tokumitsu H, Vellenga E (1997) The Ca2+/calmodulin-dependent kinase type IV is involved in the CD5- mediated signaling pathway in human T lymphocytes. J Biol Chem 272:31809-31820.
Hanissian SH, Frangakis M, Bland MM, Jawahar S, Chatila TA (1993) Expression of a Ca2+/calmodulin-dependent protein kinase, CaM kinase- Gr, in human T lymphocytes. Regulation of kinase activity by T cell receptor signaling. J Biol Chem 268:20055-20063.
Hanson PI, Schulman H (1992) Inhibitory autophosphorylation of multifunctional Ca2+/calmodulin- dependent protein kinase analyzed by site-directed mutagenesis. J Biol Chem 267:17216-17224.
Haribabu B, Hook SS, Selbert MA, Goldstein EG, Tomhave ED, Edelman AM, Snyderman R, Means AR (1995) Human calcium-calmodulin dependent protein kinase I: cDNA cloning, domain structure and activation by phosphorylation at threonine-177 by calcium-calmodulin dependent protein kinase I kinase. EMBO J 14:3679-3686.
Herdegen T, Sandkuhler J, Gass P, Kiessling M, Bravo R, Zimmermann M (1993) JUN, FOS, KROX, and CREB transcription factor proteins in the rat cortex: basal expression and induction by spreading depression and epileptic seizures. J Comp Neurol 333:271-288.
Herdegen T, Blume A, Buschmann T, Georgakopoulos E, Winter C, Schmid W, Hsieh TF, Zimmermann M, Gass P (1997) Expression of activating transcription factor-2, serum response factor and cAMP/Ca response element binding protein in the adult rat brain following generalized seizures, nerve fibre lesion and ultraviolet irradiation. Neuroscience 81:199-212.
Hughes PE, Young D, Preston KM, Yan Q, Dragunow M (1998) Differential regulation by MK801 of immediate-early genes, brain- derived neurotrophic factor and trk receptor mRNA induced by a kindling after-discharge. Brain Res Mol Brain Res 53:138-151.
Marklund U, Larsson N, Brattsand G, Osterman O, Chatila TA, Gullberg M (1994) Serine 16 of oncoprotein 18 is a major cytosolic target for the Ca2+/calmodulin-dependent kinase-Gr. Eur J Biochem 225:53-60.
Mathern GW, Leite JP, Babb TL, Pretorius JK, Kuhlman PA, Mendoza D, Fried I, Sakamoto AC, Assirati JA, Adelson PD, Peacock WJ (1996) Aberrant hippocampal mossy fiber sprouting correlates with greater NMDAR2 receptor staining. Neuroreport 7:1029-1035.
McNamara JO, Puranam RS (1998) Epilepsy genetics: an abundance of riches for biologists. Curr Biol 8:R168-R170.
Means AR, Cruzalegui F, LeMagueresse B, Needleman DS, Slaughter GR, Ono T (1991) A novel Ca2+/calmodulin-dependent protein kinase and a male germ cell- specific calmodulin-binding protein are derived from the same gene. Mol Cell Biol 11:3960-3971.
Means AR, Ribar TJ, Kane CD, Hook SS, Anderson KA (1997) Regulation and properties of the rat Ca2+/calmodulin-dependent protein kinase IV gene and its protein products. Recent Prog Horm Res 52:389-406; discus.
Meijer OC, de Kloet ER (1994) Corticosterone suppresses the expression of 5-HT1A receptor mRNA in rat dentate gyrus. Eur J Pharmacol 266:255-261.
Morimoto K, Sato K, Kashihara K, Hayabara T (1997) Increased levels of mRNA for beta- but not alpha-subunit of calmodulin kinase II following kindled seizures. Brain Res Bull 43:375-380.
Nagase T, Ishikawa K, Nakajima D (1998) Prediction of the coding sequences of unidentified human genes: The complete sequence of 100 new cDNA clones from brain which code for large proteins in vitro. DNA Res 14:141-150.
Nairn AC, Picciotto MR (1994) Calcium/calmodulin-dependent protein kinases. Semin Cancer Biol 5:295-303.
Parent JM, Lowenstein DH (1997) Mossy fiber reorganization in the epileptic hippocampus. Curr Opin Neurol 10:103-109.
Parent JM, Yu TW, Leibowitz RT, Geschwind DH, Sloviter RS, Lowenstein DH (1997) Dentate granule cell neurogenesis is increased by seizures and contributes to aberrant network reorganization in the adult rat hippocampus. J Neurosci 17:3727-3738.
Perlin JB, Churn SB, Lothman EW, DeLorenzo RJ (1992) Loss of type II calcium/calmodulin-dependent kinase activity correlates with stages of development of electrographic seizures in status epilepticus in rat. Epilepsy Res 11:111-118.
Sakagami H, Kondo H (1993) Differential expression of mRNAs encoding gamma and delta subunits of Ca2+/calmodulin-dependent protein kinase type II (CaM kinase II) in the mature and postnatally developing rat brain. Brain Res Mol Brain Res 20:51-63.
Sakagami H, Kondo H (1993) Cloning and sequencing of a gene encoding the beta polypeptide of Ca2+/calmodulin-dependent protein kinase IV and its expression confined to the mature cerebellar granule cells. Brain Res Mol Brain Res 19:215-218.
Sakagami H, Kondo H (1996) Immunohistochemical localization of Ca2+/calmodulin-dependent protein kinase type IV in the mature and developing rat retina. Brain Res 719:154-160.
Sambrook J, Fritsch EF, Maniatis T (1989) Molecular cloning: a laboratory manual. (second edition). New York: Cold Spring Harbor Laboratory Press.
Stefani A, Spadoni F, Siniscalchi A, Bernardi G (1996) Lamotrigine inhibits Ca2+ currents in cortical neurons: functional implications. Eur J Pharmacol 307:113-116.
Sun Z, Means AR (1995) An intron facilitates activation of the calspermin gene by the testis- specific transcription factor CREM tau [published erratum appears in 1995 Nov 24;270(47):28494]. J Biol Chem 270:20962-20967.
Sun Z, Means RL, LeMagueresse B, Means AR (1995) Organization and analysis of the complete rat calmodulin-dependent protein kinase IV gene. J Biol Chem 270:29507-29514.
Veeranna , Amin ND, Ahn NG, Jaffe H, Winters CA, Grant P, Pant HC (1998) Mitogen-activated protein kinases (Erk1,2) phosphorylate Lys-Ser-Pro (KSP) repeats in neurofilament proteins NF-H and NF-M. J Neurosci 18:4008-4021.
von Campe G, Spencer DD, de Lanerolle NC (1997) Morphology of dentate granule cells in the human epileptogenic hippocampus. Hippocampus 7:472-488.
von Wegerer J, Hesslinger B, Berger M, Walden J (1997) A calcium antagonistic effect of the new antiepileptic drug lamotrigine. Eur Neuropsychopharmacol 7:77-81.
Vreugdenhil E, de Jong J, Busscher JS, de Kloet ER (1996) Kainic acid-induced gene expression in the rat hippocampus is severely affected by adrenalectomy. Neurosci Lett 212:75-78.
Vreugdenhil E, de Jong J, Schaaf MJ, Meijer OC, Busscher J, Vuijst C, de Kloet ER (1996) Molecular dissection of corticosteroid action in the rat hippocampus. Application of the differential display techniques. J Mol Neurosci 7:135-146.
Wasterlain CG, Bronstein JM, Morin AM, Dwyer BE, Sankar R (1992) Translocation and autophosphorylation of brain calmodulin kinase II in status epilepticus. Epilepsy Res Suppl 9:231-238.

**Table 1**

| CARP | | CaMK-VI | |
|---|---|---|---|
| transcript species | fold induction | transcript species | fold induction |
| 8.3 kb | n.d. | 8.3 kb | 0.83 |
| 4.9 kb | 30 | 7.0 kb | 0.60 |
| 4.0 kb | n.d. | 5.0 kb | 0.60 |
| | | 4.0 kb | 0.16 |
| | | 3.4 kb | 2.50 |
| | | 1.4 kb | 0.60 |

## Claims

1. A mammalian calcium/calmodulin dependent protein kinase (CaMK-VI) which in the rat comprises the sequence 
MLELIEVNGTPGSQLSTPRSGKSPSPSPTSPGSLRKQRISQHGGSSTSLSSTKVCSSMDEN
DGPGEEESDEGFQIPATITERYKVGRTIGDGNFAVVKECIERSTAREYALKIIKKSKCRGK
EHMIQNEVSILRRVKHPNIVLLIEEMDVPTELYLVMELVKGGDLFDAITSTSKYTERDASG
MLYNLASAIKYLHSLNIVHRDIKPENLLVYEHQDGSKSLKLGDFGLATIVDGPLYTVCGTP
TYVAPEIIAETGYGLKVDIWAAGVITYILLCGFPPFRGSGDDQEVLFDQILMGQVDFPSPY
WDNVSDSAKELINMMLLVNVDQRFSAVQVLEHPWVNDDGLPENEHQLSVAGKIKKHFNTGP
KPSSTAAGVSVIAPDHGFTIKRSGSLDYYQQPGMYWIRPPLLIRRGRFSDEDATRM
 or a specific and/or active fragment or derivative thereof.

2. A mammalian protein kinase according to claim 1 which is at least partially encoded by at least a specific part of the following DNA sequence

CTCTGGCTCTTGGCTATTGTCAGGTCAAACACTAAGACTGTGTCCATGTTAGAACTCATAG
AAGTTAATGGAACCCCTGGCAGTCAGCTCTCCACTCCGCGCTCCGGCAAGTCACCAAGTCC
ATCGCCCACCAGCCCAGGAAGCCTGCGGAAGCAGAGGATCTCTCAACATGGCGGCTCCTCC
ACTTCACTTTCATCCACAAAAGTCTGCAGCTCAATGGATGAGAACGATGGCCCTGGGAAG
AAGAGTCCGACGAAGGTTTCCAGATTCCTGCCACAATAACAGAGAGATACAAAGTCGGGAG
AACAATAGGAGATGGAAATTTCGCTGTTGTCAAGGAATGTATAGAGAGGTCGACTGCTCGG
GAGTATGCCCTGAAAATTATCAAGAAAAGTAAATGCCGAGGCAAAGAACACATGATCCAAA
ACGAGGTCTCCATCTTAAGGAGAGTGAAGCATCCGAACATTGTTCTCCTGATTGAAGAGAT
GGATGTGCCGACTGAACTATATCTTGTAATGGAGTTAGTAAAGGGTGGAGACCTTTTCGAT
GCCATCACTTCCACTAGCAAATACACAGAGCGGGATGCCAGTGGGATGCTGTACAACCTGG
CCAGTGCCATTAAATACCTGCACAGCCTGAACATCGTCCATCGTGACATCAAGCCAGAGAA
CCTGCTGGTGTATGAGCACCAGGATGGCAGTAAGTCACTCAAGTTGGGTGACTTTGGCCTG
GCCACAATTGTCGATGGCCCCCTGTACACAGTCTGTGGCACCCCAACTTATGTGGCTCCAG
AAATCATTGCAGAGACTGGATATGGCCTCAAGGTGGACATCTGGGCAGCTGGCGTGATCAC
TTATATCCTGTTGTGTGGTTTCCCTCCATTCCGTGGAAGTGGGGATGACCAGGAGGTGCTT
TTTGACCAGATCTTGATGGGCCAAGTGGACTTTCCATCTCCGTATTGGGACAATGTGTCAG
ATTCTGCTAAGGAGCTCATCAACATGATGCTGTTGGTTAACGTGGACCAGAGATTTTCAGC
CGTGCAAGTCCTTGAGCACCCCTGGGTTAACGATGATGGCCTCCCAGAAAATGAACATCAG
CTGTCAGTAGCTGGCAAGATCAAGAAGCATTTCAACACGGGCCCCAAGCCGAGCAGCACGG
CAGCTGGAGTTTCTGTAATAGCACCGGACCACGGGTTTACCATCAAGAGATCAGGGTCTTT
GGACTACTACCAACAACCAGGAATGTATTGGATAAGACCACCGCTCTTGATAAGGAGAGGC

AGGTTTTCCGACGAAGACGCAACCAGGATGTGAGGGGCCGGTACAAGGCACAGCCAGCTCC
ACCAGAACTCAACTCGGAATCAGAAGACTACTCCCCAGCTCCTCTGAGACTGTCCGCTCC
CCCAATTCGCCCTTTTAATAAGACCCCTTCACTCGAAGTCCTAGCTTAACCCTTTAAGACT
CTGAGATTCCCCCTCCCCCCAAACTTCTGTAAAACAGGTTCATCTGATCTACCTAGCACTC
AGTGCTGAACGGCAGAACAGAAAGTATATTTCGGGTACCTTTGTAGCAAGTTCCCTTTACT
GAATGAGAAGGCATGTGGTGTTTGTGACAACGGTAATTTGCTGCTAATAGGGGTCCTTAAA
GGGTTGAGGCTAATTTGCGATTTTTTTCTTTTAACATAGAAGTAACGAATGTTTTCATCA
GTCAGACTAGGATCTGCAGTATGTAACATAGCACATGTTAGCCCTCTGAGTGCATAGAATT
TGCTTGAGAATCCTTGCTGGGAATTTTTCAGACCCTTGAGTGTATTCACACATATTTCTT
GATTTTACTGCAGACCAAGGGGCGTCATTGAGACCCAAAATGTCCACCCAAGAAGAGTATT
GGGGAATGACACATTGCCAATTCTTCTCAGTGGGCTTAGACAATGACGGGTTCTACAGCAT
CAGCAGATGCACCCCTCCTTCTTAGCCATCTTATGAAGAAGGAAGTCCCTTTCCCTACAT
AGTAAGCTGCAGAGCCTTCTCTGCTCTGATTGGAGGGAGTTAAGGTTTGTGGCTGCCTATC
CCAAGTGATCGACAGTGTCTCTCC.

3. A mammalian calcium/calmodulin dependent protein kinase related peptide (CARP) which in the rat has the amino acid sequence
MLELIEVNGTPGSQLSTPRSGKSPSPSPTSPGSLRKQRDLYRPLSSDDLDSGDSV
or a derivative or a specific and/or active fragment thereof.

4. A mammalian calcium/calmodulin dependent protein kinase related peptide (CARP) according to claim 3, which is at least partially encoded by at least a specific part of the following DNA sequence

GGCTATTGTCAGGTCAAACACTAAGACTGTGTCCATGTTAGAACTCATAGAAGTTAATGGA
ACCCCTGGCAGTCAGCTCTCCACTCCGCGCTCCGGCAAGTCACCAAGTCCATCGCCCACCA
GCCCAGGAAGCCTGCGGAAGCAGAGGACCTGTACCGCCCTCTCTCGTCGGATGATTTGGA
CTCAGGAGACTCAGTGTAAAAGACACAGTGGAGCGATGTGTATGGTTTGTGATGGGATCAA
GGTTTCCCATTTCCTAAGCAAGCATCTAACAGCAAAATACACAAAAGAGTTTTGCATAAGC
AATTAAATTATACAAAAGCAAGAGTGCAGACATTTTCTACTCGGCGGTATTCTTTGAAAGA
AAAAAAAAACTTTTTTAAAGCATCTCCTCCATTTGATGTAACCGAGGAGAGTTTGAATACA
ATATTTGTTCATCACTGAGGATTTTATGACTAAGGTCTGGTTCCAGATAAATTTCATATAT
GACTCCAATATTAAGTACAAGAATCACCCTAACGATCCAGCATTATGGAGTCACCATTTCT
ATAAATGAGCCTGCTTTTTAATCAGTAAGTAGATATATAAATACAATTTTTCTGTACTTAG
TATCTTTTACCTTTTTAGTTTAAATTTTTTAAAAAAATCAGACAGCTAATTAGTTATTTCA
TTGCAAAATAACATAAAAGAATCTCTTCAGGATTTTTTATTTGAGTGAATACGTCTGAAGG
GGAGTGGAGGAACTCCGTAAGAGACGTCTCCCAGAGACCAACATTAGGCAGCGAGAACGTT

TCTGGTTTTTTTTTTTTTTTTTTTTTGTTTTTGTTTTTTGCCACATGGATTTTCTTTTCCCA
GATTTTTATAAAAATAGTTTCAACTACATGGAAACATGAATAACTTGGCATTATTATTAAT
TTTATAATGGTTCCCAACAGCAACAACAGTATATATATCTTTTTCAAGTGTGTAAAGTTTT
TTGACCTAAAACAAATGGGACTTGTACTGTTTTCAAAAAATAAACTCAGTTTGAGAATTG
GAATTCTTCTACACAGAGCTTGTACGTTGGGCCTCCAGGTAGCTAAACTCCATTCTGATCA
TCCTGCATCCCGTTGTTTGGGATATCTTCACATGCAAAACTCCTTTTGTGTCAAACCGAGT
GTGTCTATGATTATAACCCACTGTTAAAATTAACCTACTAATTCTTCTCCTTTAGTCCTCT
ACTTTCAAAAGCGTGAGTCAGCTTTGCTTAAATGTAAAACAGTTATTTTTAAATCTAAAT
GTTAGAAATCAATGTGTACGGTGTATTGTATTTAGACCATACCCGTGACCGTTTCTTTTTA
CTAGCTCAAACACTAACAATTTAAGACTTACAAAGACATGGGTTTATATTCGCCAGGCTT
GACATATCTCAGTAGGGTGACAGATTTAAAATGTATGGTGCCATTTGGAGCCTTTTACTTG
AGATTTCTTTCAAATTTCACTTGAAAACATTTTGAAAATGCCTGATTCAAGATGGAGAGGA
GTCGCCTTGTCATACGGTCTCAAATGTTATTGATTTATTTCTCTCAGTAGACTGTTCGACT
AGCAATTTTTCCCATCTGGTTTATCGTTACCTGTGCTAGCATCTCACTTGACAAGTGAATA
AAATATTCCAACTGAAAAAGAAAAATAAGACCTCCAGGCAACCTACCAGCATTTGAAAAG
TTAGCCGAGTGATAATTAGTGCCTCATAACCCTGCCTGTTAGAGAGGAGAGGGCACTTCTT
TGGCGTGTTATTTGAGAACTGCAGACTGATCCCTCCATCTTGAAAGCACAGAGAATGATCA
TACCTGTTCCAGACTTCGGATTTTAAAACTGTTTCCAATAGACACGAACAGAGACTCCTGC
TGAGATCAAAGGGCCGCTTTGTCTTTAGCTGAAACAGTAAACATCTGGGAGGATGCTGTGC
TGCCTGTTGGAGTGTTACGTCTTTGTAAGATGCATTGCTTAATATAGTCCAACCCGTAAAA
GATTAGTACAGATTTGACTAACCTGTCAAAAAATGCAATGAAAGCAGTGGGTCAGATGGTC
CAGAACAAAACATAAATTCCAGTAATTTCAACTCCCCTATGAAGTCAATCTTATGGACGAC
TACTGCTAGGAGAAACCTAATAAATGTTATCTGCTATAAAAATCGTGGCTTTGTTTAGACA
CAGCATAGAGTAGACTTGGACTAATTGCCAGTAGGTATTGTGGCCAACTTTCCATTCAAGC
AGTTCCAGAGAGATGCAATCCCATGGCATTTCATCCTGGAACATACGATTTTTATTAAAGG
ATTGTTCAAGGGCCATATATCTCATTTACTTTTGAAACTAAAGTCCCTCTGAGATTGAAAC
AACACACCCTGAGATCTCTTTCTATGCACGGAATAATTCTATTATCCAAAAGCAGAGTTTT
TGAACATCCAAGCAGCCCACCAGGTCATTAGTAAAGACCCAATGGGCAATTAGCAGTCACT
CAAGACGTGGCTGCTTTTAGAATCCTTCTAACATCATTAGTCGCTGAACTCTAAAAATGAC
CTTCTGAGGAAAGAGCTCCAGCCAACAAAAGCCACATGCTTTTCTCTCACTGATAAAGCCT
CTGCTGTGTTGACCTTTCAGGCCTTTGCATGTATGGTTACATTTTTTTAGACATACCTAGA
AAGAAAGATGCTATAAAGAGTCCCCTGCTTTCAGCATTTTATGAGCTAAAACCCTAGTGAT
GCACAAAATGTGTTGCTCTGCGTGAACTCCGAAGCCATGTGGGAAAGTCATTGTCATTTCA
GCAGAAGGTCTGTACTCTTGTAGGTTTCTAAACCGGAAGTTTGTGCTCCACCTATATGTAC
GCATGCCTGCAATGCACACATGAGGTCACCTTAACATGTCAAGATCTGATCCCGTTGTCTG
CCAACCCCAGAGCAGCTCTGACACCTATGCTCCCCTGCCAAGCAGCGAGGGCACTCGCCCT

CCCCTTCCTTGCAGCCGCACCGAGCTGTACTTTTGTTTCCATTTAAAAGAACTGAATTCCA
AAAGCCTCGTTTTAAAAAAGCAATTGGGAATGAAAATACATAAGAATAATCCTAAAAGATA
AATCCATATATTTAACCTGTTGAACTACTAGGTTTTTTTTTCTTTTCCTGGTGGCCATAT
ATCTATACAGGTGTGTAGGTGTGCCTA
CATGAATATCTGTGTTTGTATATAGAACATGAAGAATAAAGGATGATATTTGTGATATGGT
CTTATAAGTTACTGGCTCCTGTAGAATTAACTGTGAAGCTTTATGTGATAAAGTTGTGACG
TATGTATTAGTTGGCACATGTTTAAGCTCAAAACAAGTTGGCTAAATTATCATGAAGCAGT
ACCAACTGTTCACGGGTACGAATTTATCCAAGACCGATTGGAATCCAGACAAACAAAAAAA
TTGAAGTCCAAGCAAATCCAATCTTACAGTGATTGATAAATGTAAATACTCATTGATGTTT
TCACTGTAAATTCTTGATGCTCAGCCAGTAATCTGACAGAATGCAGTGACACTTTCCTGTT
GTGTGTGTACTGATGACTACAGCGATTATCAATGGAAATTCACCAAACAAACTGATTTCT
AGACTTGTGGACAGTTGAGTGTTTGTGCTGAAATAAAAGTAGAAAAGGTCAAAGTAAATTC
GGTTTGAAGCCTTGTTATGAAGCCAGACAACATGTAAGGCCATGCTGTGTGTCCTTTTTAT
AAACTG[?]TTTGGAGACACTTGCTGTGTGTGTGCATGTGTGTCTACATGAGTGTATACACAC
TTGAAAATGCTTATTGACTCAAGTTCACTGTCTGTATACATCTGTGCCTATCTGCATTGGT
CTGGCCAGCGCCTCTAACTGTGCATGGTAGTGACAGCCCTGTGTGATTGGATGAACCCACA
GAAAAATAGTGTGGAAATCAGAGCTGCGTCAAACTCATTATAACAATTGCCTGATATAGCA
TATTAAATTGTCAGCTGAAAAACGTTTGTAATGTTGTGCCTGTAACATGTGTAGGGAAAAC
TGGAAAATCTGAGGCCCTCACTATAATTTTAAGGGTGCATGACATGTGGTGTCTTTAAAGA
TGTATCTTATTGTAATAAAAGGTGATGGATAAATTCTGAGGTAGCGTAAGTGAAGCATGAA
ACGCTAGGTGTTTGTATTATATATGGCCACACAATATTTTTGAAGGCAGGGTAGTAAATGT
TTGAAGGTTAATAGGCAAAGAATATGGAAGTTAATTTTACTGTGATGTGTTTTTGAGGTGT
TTTACACATTGCCAGACAAGGAACTTAGTTCCTGTTTCCCCCCTGGGACTCTTTGAAACAC
TATGAATGTTACAAAGTTGGATTTTCTTTATAAAGGTCGCAAACCATTTGCAGTTGGAGTT
GCTTGGATGTATAGTCTCCTCTTTGGGGAAAAAAACCACTTTGGTATTAAAATGGTTTAAG
CACTAATACATTGGGAAATACGTCGTCTGAGGCCATCTGCAGACATCTGTAGAGATGAGAA
CTAATGAAATGTGTTACTGAACTACCCAGTTTTGATAGCTTCCATGCCTCTCTGCTGTAGT
CTCCTCGAAAATGCTGTACCTTGTGTTTCACATGCAACCATGTTACTCTCTCCCGAACTTA
CCTTTGTGACACTTAGCAGCTTAGCCAAACCACGTAATAAACAGGAAGAACTGCCAAAAAA
AAAAAAAAAAAAAAAAAAAAA.

5. A recombinant and/or isolated nucleic acid molecule encoding at least a specific and/or active part of a mammalian calcium/calmodulin dependent protein kinase (CaMK-VI).

6. A recombinant and/or isolated nucleic acid molecule encoding at least a specific and/or active part of a mammalian calcium/calmodulin dependent protein kinase related peptide (CARP).

7. A cloning and/or expression vector comprising an isolated and/or recombinant nucleic acid molecule according to claim 5 or 6.

8. A mammalian host cell comprising a recombinant and/or isolated nucleic acid according to claim 5 or 6 or a vector according to claim 7.

9. A mammalian calcium/calmodulin dependent protein kinase related peptide (CARP) for use as a pharmaceutical.

10. Use of a mammalian calcium/calmodulin dependent protein kinase related peptide (CARP) in the preparation of a medicament for the treatment and/or prevention of seizures and/on damage to the central nervous system, in particular the hippocampus.

11. A method for down regulating calcium/calmodulin dependent protein kinase activity in cells comprising providing said cells with a mammalian calcium/calmodulin dependent protein kinase related peptide (CARP) according to claim 3 or 4.


SEQUENCE LISTING

(1) GENERAL INFORMATION:

(i) APPLICANT:
(A) NAME: Division of Medical Pharmacology
Leiden/Amsterdam Centre for Drug Research
(B) STREET: Wassenaarseweg 72
(C) CITY: Leiden
(D) STATE: Zuid-Holland
(E) COUNTRY: the Netherlands
(F) POSTAL CODE (ZIP): 2333 AL

(ii) TITLE OF INVENTION: A novel calcium/calmodulin dependent kinase
and an alternative splice product thereof.

(iii) NUMBER OF SEQUENCES: 20

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98202654.4

(2) INFORMATION FOR SEQ ID NO: 1:

(i) SEQUENCE CHARACTERISTICS:
(A) LENGTH: 419 amino acids
(B) TYPE: amino acid
(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO: 1:

Met Leu Glu Leu Ile Glu Val Asn Gly Thr Pro Gly Ser Gln Leu Ser
1               5                   10                  15

Thr Pro Arg Ser Gly Lys Ser Pro Ser Pro Ser Pro Thr Ser Pro Gly
            20                  25                  30

Ser Leu Arg Lys Gln Arg Ile Ser Gln His Gly Gly Ser Ser Thr Ser
        35                  40                  45

Leu Ser Ser Thr Lys Val Cys Ser Ser Met Asp Glu Asn Asp Gly Pro
    50                  55                  60

Gly Glu Glu Glu Ser Asp Glu Gly Phe Gln Ile Pro Ala Thr Ile Thr

65                  70                  75                  80

Glu Arg Tyr Lys Val Gly Arg Thr Ile Gly Asp Gly Asn Phe Ala Val
                85                  90                  95

Val Lys Glu Cys Ile Glu Arg Ser Thr Ala Arg Glu Tyr Ala Leu Lys
            100                 105                 110

Ile Ile Lys Lys Ser Lys Cys Arg Gly Lys Glu His Met Ile Gln Asn
        115                 120                 125

Glu Val Ser Ile Leu Arg Arg Val Lys His Pro Asn Ile Val Leu Leu
    130                 135                 140

Ile Glu Glu Met Asp Val Pro Thr Glu Leu Tyr Leu Val Met Glu Leu
145                 150                 155                 160

Val Lys Gly Gly Asp Leu Phe Asp Ala Ile Thr Ser Thr Ser Lys Tyr
                165                 170                 175

Thr Glu Arg Asp Ala Ser Gly Met Leu Tyr Asn Leu Ala Ser Ala Ile
            180                 185                 190

Lys Tyr Leu Ser Leu Asn Ile Val His Arg Asp Ile Lys Pro Glu Asn
        195                 200                 205

Leu Leu Val Tyr Glu His Gln Asp Gly Ser Lys Ser Leu Lys Leu Gly
    210                 215                 220

Asp Phe Gly Leu Ala Thr Ile Val Asp Gly Pro Leu Tyr Thr Val Cys
225                 230                 235                 240

Gly Thr Pro Thr Tyr Val Ala Pro Glu Ile Ile Ala Glu Thr Gly Tyr
                245                 250                 255

Gly Leu Lys Val Ile Trp Ala Ala Gly Val Ile Thr Tyr Ile Leu Leu
            260                 265                 270

Cys Gly Phe Pro Pro Phe Arg Gly Ser Gly Asp Asp Gln Glu Val Leu
        275                 280                 285

Phe Asp Gln Ile Leu Met Gly Gln Val Asp Phe Pro Ser Pro Tyr Trp
    290                 295                 300

Asp Asn Val Ser Asp Ser Ala Lys Glu Leu Ile Asn Met Met Leu Leu
305                 310                 315                 320

Val Asn Val Asp Gln Arg Phe Ser Ala Val Gln Val Leu Glu His Pro
                325                 330                 335

Trp Val Asn Asp Asp Gly Leu Pro Glu Asn Glu His Gln Leu Ser Val
            340                 345                 350

Ala Gly Lys Ile Lys Lys His Phe Asn Thr Gly Pro Lys Pro Ser Ser
        355                 360                 365

Thr Ala Ala Gly Val Ser Val Ile Ala Pro Asp His Gly Phe Thr Ile
    370                 375                 380

Lys Arg Ser Ser Leu Asp Tyr Tyr Gln Gln Pro Gly Met Tyr Trp Ile
385                390                395                400

Arg Pro Pro Leu Leu Ile Arg Arg Gly Arg Phe Ser Asp Glu Asp Ala
                405                410                415

Thr Arg Met

(2) INFORMATION FOR SEQ ID NO: 2:

(i) SEQUENCE CHARACTERISTICS:
(A) LENGTH: 2098 base pairs
(B) TYPE: nucleic acid
(C) STRANDEDNESS: unknown
(D) TOPOLOGY: unknown

(ii) MOLECULE TYPE: cDNA

(iii) HYPOTHETICAL: NO

(xi) SEQUENCE DESCRIPTION: SEQ ID NO: 2:

CTCTGGCTCT TGGCTATTGT CAGGTCAAAC ACTAAGACTG TGTCCATGTT
AGAACTCATA     60

GAAGTTAATG GAACCCCTGG CAGTCAGCTC TCCACTCCGC GCTCCGGCAA
GTCACCAAGT     120

CCATCGCCCA CCAGCCCAGG AAGCCTGCGG AAGCAGAGGA TCTCTCAACA
TGGCGGCTCC     180

TCCACTTCAC TTTCATCCAC AAAAGTCTGC AGCTCAATGG ATGAGAACGA
TGGCCCTGGG     240

GAAGAAGAGT CCGACGAAGG TTTCCAGATT CCTGCCACAA TAACAGAGAG
ATACAAAGTC     300

GGGAGAACAA TAGGAGATGG AAATTTCGCT GTTGTCAAGG AATGTATAGA
GAGGTCGACT     360

GCTCGGGAGT ATGCCCTGAA AATTATCAAG AAAAGTAAAT GCCGAGGCAA
AGAACACATG     420

ATCCAAAACG AGGTCTCCAT CTTAAGGAGA GTGAAGCATC CGAACATTGT
TCTCCTGATT     480

GAAGAGATGG ATGTGCCGAC TGAACTATAT CTTGTAATGG AGTTAGTAAA
GGGTGGAGAC     540

CTTTTCGATG CCATCACTTC CACTAGCAAA TACACAGAGC GGGATGCCAG
TGGGATGCTG     600

TACAACCTGG CCAGTGCCAT TAAATACCTG CACAGCCTGA ACATCGTCCA
TCGTGACATC 660

AAGCCAGAGA ACCTGCTGGT GTATGAGCAC CAGGATGGCA GTAAGTCACT
CAAGTTGGGT 720

GACTTTGGCC TGGCCACAAT TGTCGATGGC CCCCTGTACA CAGTCTGTGG
CACCCCAACT 780

TATGTGGCTC CAGAAATCAT TGCAGAGACT GGATATGGCC TCAAGGTGGA
CATCTGGGCA 840

GCTGGCGTGA TCACTTATAT CCTGTTGTGT GGTTTCCCTC CATTCCGTGG
AAGTGGGGAT 900

GACCAGGAGG TGCTTTTTGA CCAGATCTTG ATGGGCCAAG TGGACTTTCC
ATCTCCGTAT 960

TGGGACAATG TGTCAGATTC TGCTAAGGAG CTCATCAACA TGATGCTGTT
GGTTAACGTG 1020

GACCAGAGAT TTTCAGCCGT GCAAGTCCTT GAGCACCCCT GGGTTAACGA
TGATGGCCTC 1080

CCAGAAAATG AACATCAGCT GTCAGTAGCT GGCAAGATCA AGAAGCATTT
CAACACGGGC 1140

CCCAAGCCGA GCAGCACGGC AGCTGGAGTT TCTGTAATAG CACCGGACCA
CGGGTTTACC 1200

ATCAAGAGAT CAGGGTCTTT GGACTACTAC CAACAACCAG GAATGTATTG
GATAAGACCA 1260

CCGCTCTTGA TAAGGAGAGG CAGGTTTTCC GACGAAGACG CAACCAGGAT
GTGAGGGGCC 1320

GGTACAAGGC ACAGCCAGCT CCACCAGAAC TCAACTCGGA ATCAGAAGAC
TACTCCCCCA 1380

GCTCCTCTGA GACTGTCCGC TCCCCCAATT CGCCCTTTTA ATAAGACCCC
TTCACTCGAA 1440

GTCCTAGCTT AACCCTTTAA GACTCTGAGA TTCCCCCTCC CCCCAAACTT
CTGTAAAACA 1500

GGTTCATCTG ATCTACCTAG CACTCAGTGC TGAACGGCAG AACAGAAAGT
ATATTTCGGG 1560

TACCTTTGTA GCAAGTTCCC TTTACTGAAT GAGAAGGCAT GTGGTGTTTG
TGACAACGGT 1620

AATTTGCTGC TAATAGGGGT CCTTAAAGGG TTGAGGCTAA TTTGCGATTT
TTTTTCTTTT 1680

AACATAGAAG TAACGAATGT TTTCATCAGT CAGACTAGGA TCTGCAGTAT
GTAACATAGC 1740

ACATGTTAGC CCTCTGAGTG CATAGAATTT GCTTGAGAAT CCTTGCTGGG
GAATTTTTCA 1800

GACCCTTGAG TGTATTCACA CATATTTCTT GATTTTACTG CAGACCAAGG
GGCGTCATTG 1860

AGACCCAAAA TGTCCACCCA AGAAGAGTAT TGGGGAATGA CACATTGCCA
ATTCTTCTCA 1920

GTGGGCTTAG ACAATGACGG GTTCTACAGC ATCAGCAGAT GCACCCCTCC
TTCTTAGCCA 1980

TCTTATGAAG AAGGGAAGTC CCTTTCCCTA CATAGTAAGC TGCAGAGCCT
TCTCTGCTCT 2040

GATTGGAGGG AGTTAAGGTT TGTGGCTGCC TATCCCAAGT GATCGACAGT GTCTCTCC
2098

(2) INFORMATION FOR SEQ ID NO: 3:

(i) SEQUENCE CHARACTERISTICS:
(A) LENGTH: 55 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 3:

Met Leu Glu Leu Ile Glu Val Asn Gly Thr Pro Gly Ser Gln Leu Ser
1               5                   10                  15

Thr Pro Arg Ser Gly Lys Ser Pro Ser Pro Ser Pro Thr Ser Pro Gly
            20                  25                  30

Ser Leu Arg Lys Gln Arg Asp Leu Tyr Arg Pro Leu Ser Ser Asp Asp
        35                  40                  45

Leu Asp Ser Gly Asp Ser Val
    50                  55

(2) INFORMATION FOR SEQ ID NO: 4:

(i) SEQUENCE CHARACTERISTICS:
(A) LENGTH: 56 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 4:

Met Leu Glu Leu Ile Glu Val Asn Gly Thr Pro Gly Ser Gln Leu Ser
1               5                   10                  15

Thr Pro Arg Ser Gly Lys Ser Pro Ser Pro Ser Pro Thr Ser Pro Gly
            20                  25                  30

Ser Leu Arg Lys Gln Arg Asp Leu Tyr Arg Pro Leu Ser Ser Asp Asp
        35                  40                  45

Leu Asp Ser Val Gly Asp Ser Val
    50                  55

(2) INFORMATION FOR SEQ ID NO: 5:

(i) SEQUENCE CHARACTERISTICS:
(A) LENGTH: 4746 base pairs
(B) TYPE: nucleic acid
(C) STRANDEDNESS: unknown
(D) TOPOLOGY: unknown

(ii) MOLECULE TYPE: cDNA

(iii) HYPOTHETICAL: NO

(xi) SEQUENCE DESCRIPTION: SEQ ID NO: 5:

GGCTATTGTC AGGTCAAACA CTAAGACTGT GTCCATGTTA GAACTCATAG
AAGTTAATGG    60

AACCCCTGGC AGTCAGCTCT CCACTCCGCG CTCCGGCAAG TCACCAAGTC
CATCGCCCAC    120

CAGCCCAGGA AGCCTGCGGA AGCAGAGGGA CCTGTACCGC CCTCTCTCGT
CGGATGATTT    180

GGACTCAGGA GACTCAGTGT AAAAGACACA GTGGAGCGAT GTGTATGGTT
TGTGATGGGA    240

TCAAGGTTTC CCATTTCCTA AGCAAGCATC TAACAGCAAA ATACACAAAA
GAGTTTTGCA    300

TAAGCAATTA AATTATACAA AAGCAAGAGT GCAGACATTT TCTACTCGGC
GGTATTCTTT    360

GAAAGAAAAA AAAAACTTTT TTAAAGCATC TCCTCCATTT GATGTAACCG
AGGAGAGTTT    420

GAATACAATA TTTGTTCATC ACTGAGGATT TTATGACTAA GGTCTGGTTC
CAGATAAATT    480

TCATATATGA CTCCAATATT AAGTACAAGA ATCACCCTAA CGATCCAGCA
TTATGGAGTC 540

ACCATTTCTA TAAATGAGCC TGCTTTTTAA TCAGTAAGTA GATATATAAA
TACAATTTTT 600

CTGTACTTAG TATCTTTTAC CTTTTTAGTT TAAATTTTTT AAAAAAATCA
GACAGCTAAT 660

TAGTTATTTC ATTGCAAAAT AACATAAAAG AATCTCTTCA GGATTTTTTA
TTTGAGTGAA 720

TACGTCTGAA GGGGAGTGGA GGAACTCCGT AAGAGACGTC TCCCAGAGAC
CAACATTAGG 780

CAGCGAGAAC GTTTCTGGTT TTTTTTTTTT TTTTTTTTGT TTTTGTTTTT TGCCACATGG
840

ATTTTCTTTT CCCAGATTTT TATAAAAATA GTTTCAACTA CATGGAAACA
TGAATAACTT 900

GGCATTATTA TTAATTTTAT AATGGTTCCC AACAGCAACA ACAGTATATA
TATCTTTTTC 960

AAGTGTGTAA AGTTTTTTGA CCTAAAACAA ATGGGACTTG TACTGTTTTT
CAAAAAATAA 1020

ACTCAGTTTG AGAATTGGAA TTCTTCTACA CAGAGCTTGT ACGTTGGGCC
TCCAGGTAGC 1080

TAAACTCCAT TCTGATCATC CTGCATCCCG TTGTTTGGGA TATCTTCACA
TGCAAAACTC 1140

CTTTTGTGTC AAACCGAGTG TGTCTATGAT TATAACCCAC TGTTAAAATT
AACCTACTAA 1200

TTCTTCTCCT TTAGTCCTCT ACTTTCAAAA GCGTGAGTCA GCTTTGCTTA
AATGTAAAAC 1260

AGTTATTTTT TAAATCTAAA TGTTAGAAAT CAATGTGTAC GGTGTATTGT
ATTTAGACCA 1320

TACCCGTGAC CGTTTCTTTT TACTAGCTCA AACACTAACA ATTTAAGACT
TACAAAGACA 1380

TGGGTTTTAT ATTCGCCAGG CTTGACATAT CTCAGTAGGG TGACAGATTT
AAAATGTATG 1440

GTGCCATTTG GAGCCTTTTA CTTGAGATTT CTTTCAAATT TCACTTGAAA
ACATTTTGAA 1500

AATGCCTGAT TCAAGATGGA GAGGAGTCGC CTTGTCATAC GGTCTCAAAT
GTTATTGATT 1560

TATTTCTCTC AGTAGACTGT TCGACTAGCA ATTTTTCCCA TCTGGTTTAT CGTTACCTGT
1620

GCTAGCATCT CACTTGACAA GTGAATAAAA TATTCCAACT GAAAAAAGAA
AAATAAGACC 1680

TCCAGGCAAC CTACCAGCAT TTGAAAAGTT AGCCGAGTGA TAATTAGTGC
CTCATAACCC 1740

TGCCTGTTAG AGAGGAGAGG GCACTTCTTT GGCGTGTTAT TTGAGAACTG
CAGACTGATC 1800

CCTCCATCTT GAAAGCACAG AGAATGATCA TACCTGTTCC AGACTTCGGA
TTTTAAAACT 1860

GTTTCCAATA GACACGAACA GAGACTCCTG CTGAGATCAA AGGGCCGCTT
TGTCTTTAGC 1920

TGAAACAGTA AACATCTGGG AGGATGCTGT GCTGCCTGTT GGAGTGTTAC
GTCTTTGTAA 1980

GATGCATTGC TTAATATAGT CCAACCCGTA AAAGATTAGT ACAGATTTGA
CTAACCTGTC 2040

AAAAAATGCA ATGAAAGCAG TGGGTCAGAT GGTCCAGAAC AAAACATAAA
TTCCAGTAAT 2100

TTCAACTCCC CTATGAAGTC AATCTTATGG ACGACTACTG CTAGGAGAAA
CCTAATAAAT 2160

GTTATCTGCT ATAAAAATCG TGGCTTTGTT TAGACACAGC ATAGAGTAGA
CTTGGACTAA 2220

TTGCCAGTAG GTATTGTGGC CAACTTTCCA TTCAAGCAGT TCCAGAGAGA
TGCAATCCCA 2280

TGGCATTTCA TCCTGGAACA TACGATTTTT ATTAAAGGAT TGTTCAAGGG
CCATATATCT 2340

CATTTACTTT TGAAACTAAA GTCCCTCTGA GATTGAAACA ACACACCCTG
AGATCTCTTT 2400

CTATGCACGG AATAATTCTA TTATCCAAAA GCAGAGTTTT TGAACATCCA
AGCAGCCCAC 2460

CAGGTCATTA GTAAAGACCC AATGGGCAAT TAGCAGTCAC TCAAGACGTG
GCTGCTTTTA 2520

GAATCCTTCT AACATCATTA GTCGCTGAAC TCTAAAAATG ACCTTCTGAG
GAAAGAGCTC 2580

CAGCCAACAA AAGCCACATG CTTTTCTCTC ACTGATAAAG CCTCTGCTGT
GTTGACCTTT 2640

CAGGCCTTTG CATGTATGGT TACATTTTTT TAGACATACC TAGAAAGAAA
GATGCTATAA 2700

AGAGTCCCCT GCTTTCAGCA TTTTATGAGC TAAAACCCTA GTGATGCACA
AAATGTGTTG 2760

CTCTGCGTGA ACTCCGAAGC CATGTGGGAA AGTCATTGTC ATTTCAGCAG
AAGGTCTGTA 2820

CTCTTGTAGG TTTCTAAACC GGAAGTTTGT GCTCCACCTA TATGTACGCA
TGCCTGCAAT 2880

GCACACATGA GGTCACCTTA ACATGTCAAG ATCTGATCCC GTTGTCTGCC
AACCCCAGAG 2940

CAGCTCTGAC ACCTATGCTC CCCTGCCAAG CAGCGAGGGC ACTCGCCCTC
CCCTTCCTTG 3000

CAGCCGCACC GAGCTGTACT TTTGTTTCCA TTTAAAAGAA CTGAATTCCA
AAAGCCTCGT 3060

TTTAAAAAAG CAATTGGGAA TGAAAATACA TAAGAATAAT CCTAAAAGAT
AAATCCATAT 3120

ATTTAACCTG TTGAACTACT AGGTTTTTTT TTTCTTTTCC TGGTGGCCAT ATATCTATAC
3180

AGGTGTGTAG GTGTGCCTAC ATGAATATCT GTGTTTGTAT ATAGAACATG
AAGAATAAAG 3240

GATGATATTT GTGATATGGT CTTATAAGTT ACTGGCTCCT GTAGAATTAA
CTGTGAAGCT 3300

TTATGTGATA AAGTTGTGAC GTATGTATTA GTTGGCACAT GTTTAAGCTC
AAAACAAGTT 3360

GGCTAAATTA TCATGAAGCA GTACCAACTG TTCACGGGTA CGAATTTATC
CAAGACCGAT 3420

TGGAATCCAG ACAAACAAAA AAATTGAAGT CCAAGCAAAT CCAATCTTAC
AGTGATTGAT 3480

AAATGTAAAT ACTCATTGAT GTTTTCACTG TAAATTCTTG ATGCTCAGCC
AGTAATCTGA 3540

CAGAATGCAG TGACACTTTC CTGTTGTGTG TGTACTGATG ACTACAGCGA
TTATCAATGG 3600

GAAATTCACC AAACAAACTG ATTTCTAGAC TTGTGGACAG TTGAGTGTTT
GTGCTGAAAT 3660

AAAAGTAGAA AAGGTCAAAG TAAATTCGGT TTGAAGCCTT GTTATGAAGC
CAGACAACAT 3720

GTAAGGCCAT GCTGTGTGTC CTTTTTATAA ACTGTTTTGG AGACACTTGC
TGTGTGTGTG 3780

CATGTGTGTC TACATGAGTG TATACACACT TGAAAATGCT TATTGACTCA
AGTTCACTGT 3840

CTGTATACAT CTGTGCCTAT CTGCATTGGT CTGGCCAGCG CCTCTAACTG
TGCATGGTAG 3900

TGACAGCCCT GTGTGATTGG ATGAACCCAC AGAAAAATAG TGTGGAAATC
AGAGCTGCGT 3960

CAAACTCATT ATAACAATTG CCTGATATAG CATATTAAAT TGTCAGCTGA
AAAACGTTTG 4020

TAATGTTGTG CCTGTAACAT GTGTAGGGAA AACTGGAAAA TCTGAGGCCC
TCACTATAAT 4080

TTTAAGGGTG CATGACATGT GGTGTCTTTA AAGATGTATC TTATTGTAAT
AAAAGGTGAT 4140

GGATAAATTC TGAGGTAGCG TAAGTGAAGC ATGAAACGCT AGGTGTTTGT
ATTATATATG 4200

GCCACACAAT ATTTTTGAAG GCAGGGTAGT AAATGTTTGA AGGTTAATAG
GCAAAGAATA 4260

TGGAAGTTAA TTTTACTGTG ATGTGTTTTT GAGGTGTTTT ACACATTGCC
AGACAAGGAA 4320

CTTAGTTCCT GTTTCCCCCC TGGGACTCTT TGAAACACTA TGAATGTTAC
AAAGTTGGAT 4380

TTTCTTTATA AAGGTCGCAA ACCATTTGCA GTTGGAGTTG CTTGGATGTA
TAGTCTCCTC 4440

TTTGGGGAAA AAAACCACTT TGGTATTAAA ATGGTTTAAG CACTAATACA
TTGGGAAATA 4500

CGTCGTCTGA GGCCATCTGC AGACATCTGT AGAGATGAGA ACTAATGAAA
TGTGTTACTG 4560

AACTACCCAG TTTTGATAGC TTCCATGCCT CTCTGCTGTA GTCTCCTCGA
AAATGCTGTA 4620

CCTTGTGTTT CACATGCAAC CATGTTACTC TCTCCCGAAC TTACCTTTGT
GACACTTAGC 4680

AGCTTAGCCA AACCACGTAA TAAACAGGAA GAACTGCCAA AAAAAAAAAA
AAAAAAAAAA 4740

AAAAAA 4746

(2) INFORMATION FOR SEQ ID NO: 6:

(i) SEQUENCE CHARACTERISTICS:
(A) LENGTH: 24 amino acids
(B) TYPE: amino acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 6:

Cys Thr Cys Thr Gly Gly Cys Thr Cys Thr Thr Gly Gly Cys Thr Ala
1               5                   10                  15

Thr Thr Gly Thr Cys Ala Gly Gly
            20

(2) INFORMATION FOR SEQ ID NO: 7:

(i) SEQUENCE CHARACTERISTICS:
(A) LENGTH: 24 amino acids
(B) TYPE: amino acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 7:

Gly Gly Ala Gly Ala Gly Ala Cys Ala Cys Thr Gly Thr Cys Gly Ala
1               5                   10                  15

Thr Cys Ala Cys Thr Thr Gly Gly
            20

(2) INFORMATION FOR SEQ ID NO: 8:

(i) SEQUENCE CHARACTERISTICS:
(A) LENGTH: 18 amino acids
(B) TYPE: amino acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 8:

Cys Ala Thr Gly Gly Ala Ala Ala Cys Ala Thr Gly Ala Ala Thr Ala
1               5                   10                  15

Ala Cys

(2) INFORMATION FOR SEQ ID NO: 9:

(i) SEQUENCE CHARACTERISTICS:

(A) LENGTH: 18 amino acids
(B) TYPE: amino acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 9:

Cys Gly Gly Gly Ala Gly Ala Gly Ala Gly Thr Ala Ala Cys Ala Thr
1               5                   10                  15

Gly Gly

(2) INFORMATION FOR SEQ ID NO: 10:

(i) SEQUENCE CHARACTERISTICS:
(A) LENGTH: 20 amino acids
(B) TYPE: amino acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 10:

Ala Gly Thr Cys Thr Gly Thr Gly Gly Cys Ala Cys Cys Cys Cys Ala
1               5                   10                  15

Ala Cys Thr Thr
            20

(2) INFORMATION FOR SEQ ID NO: 11:

(i) SEQUENCE CHARACTERISTICS:
(A) LENGTH: 22 amino acids
(B) TYPE: amino acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 11:

Gly Ala Cys Ala Thr Thr Gly Thr Thr Gly Cys Cys Ala Thr Cys Ala
1               5                   10                  15

Ala Cys Gly Ala Cys Cys
        20

(2) INFORMATION FOR SEQ ID NO: 12:

(i) SEQUENCE CHARACTERISTICS:
(A) LENGTH: 22 amino acids
(B) TYPE: amino acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 12:

Thr Gly Cys Ala Thr Thr Gly Cys Thr Gly Ala Cys Ala Ala Thr Cys
1               5                   10                  15

Thr Thr Gly Ala Gly Gly
        20

(2) INFORMATION FOR SEQ ID NO: 13:

(i) SEQUENCE CHARACTERISTICS:
(A) LENGTH: 9 amino acids
(B) TYPE: amino acid
(C) STRANDEDNESS: unknown
(D) TOPOLOGY: unknown

(ii) MOLECULE TYPE: other nucleic acid

(iii) HYPOTHETICAL: NO

(xi) SEQUENCE DESCRIPTION: SEQ ID NO: 13:

Gly Thr Pro Xaa Xaa Xaa Ala Pro Glu
1               5

(2) INFORMATION FOR SEQ ID NO: 14:

(i) SEQUENCE CHARACTERISTICS:
(A) LENGTH: 267 base pairs
(B) TYPE: nucleic acid
(C) STRANDEDNESS: unknown
(D) TOPOLOGY: unknown

(ii) MOLECULE TYPE: cDNA

(iii) HYPOTHETICAL: NO

(xi) SEQUENCE DESCRIPTION: SEQ ID NO: 14:

GCTCTGGCTC TTGGCTATTG TCAGGTCAAA CACTAAGACT GTGTCCATGT
TAGAACTCAT 60

AGAAGTTAAT GGAACCCCTG GCAGTCAGCT CTCCACTCCG CGCTCCGGCA
AGTCACCAAG 120

TCCATCGCCC ACCAGCCCAG GAAGCCTGCG GAAGCAGAGG ATCTCTCAAC
ATGGCGGCTC 180

CTCCACTTCA CTTTCATCCA CAAAAGTCTG CAGCTCAATG GATGAGAACG
ATGGCCCTGG 240

GGAAGAAGAG TCCGACGAAG GTTTCCA 267

(2) INFORMATION FOR SEQ ID NO: 15:

(i) SEQUENCE CHARACTERISTICS:
(A) LENGTH: 255 base pairs
(B) TYPE: nucleic acid
(C) STRANDEDNESS: unknown
(D) TOPOLOGY: unknown

(ii) MOLECULE TYPE: cDNA

(iii) HYPOTHETICAL: NO

(xi) SEQUENCE DESCRIPTION: SEQ ID NO: 15:

GGCTATTGTC AGGTCAAACA CTAAGACTGT GTCCATGTTA GAACTCATAG
AAGTTAATGG 60

AACCCCTGGC AGTCAGCTCT CCACTCCGCG CTCCGGCAAG TCACCAAGTC
CATCGCCCAC 120

CAGCCCAGGA AGCCTGCGGA AGCAGAGGGA CCTGTACCGC CCTCTCTCGT
CGGATGATTT 180

GGACTCAGGA GACTCAGTGT AAAAGACACA GTGGAGCGAT GTGTATGGTT
TGTGATGGGA 240

TCAAGGTTTC CCATT 255

(2) INFORMATION FOR SEQ ID NO: 16:

(i) SEQUENCE CHARACTERISTICS:

(A) LENGTH: 74 amino acids
(B) TYPE: amino acid
(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO: 16:

Met Leu Glu Leu Ile Glu Val Asn Gly Thr Pro Gly Ser Gln Leu Ser
1               5                   10                  15

Thr Pro Arg Ser Gly Lys Ser Pro Ser Pro Ser Pro Thr Ser Pro Gly
            20                  25                  30

Ser Leu Arg Lys Gln Arg Ile Ser Gln His Gly Gly Ser Ser Thr Ser
        35                  40                  45

Leu Ser Ser Thr Lys Val Cys Ser Ser Met Asp Glu Asn Asp Gly Pro
    50                  55                  60

Gly Glu Glu Glu Ser Asp Glu Gly Phe Gln
65                  70

(2) INFORMATION FOR SEQ ID NO: 17:

(i) SEQUENCE CHARACTERISTICS:
(A) LENGTH: 55 amino acids
(B) TYPE: amino acid
(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO: 17:

Met Leu Glu Leu Ile Glu Val Asn Gly Thr Pro Gly Ser Gln Leu Ser
1               5                   10                  15

Thr Pro Arg Ser Gly Lys Ser Pro Ser Pro Ser Pro Thr Ser Pro Gly
            20                  25                  30

Ser Leu Arg Lys Gln Arg Asp Leu Tyr Arg Pro Leu Ser Ser Asp Asp
        35                  40                  45

Leu Asp Ser Gly Asp Ser Val
    50                  55

(2) INFORMATION FOR SEQ ID NO: 18:

(i) SEQUENCE CHARACTERISTICS:
(A) LENGTH: 72 amino acids
(B) TYPE: amino acid
(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO: 18:

Thr Pro Gln Lys Thr Ser Ala Lys Ser Pro Gly Pro Met Arg Arg Ser
1               5                   10                  15

Lys Ser Pro Ala Asp Ser Ala Asn Gly Thr Ser Ser Ser Gln Leu Ser
            20                  25                  30

Thr Pro Lys Ser Lys Gln Ser Pro Ile Ser Thr Pro Thr Ser Pro Gly
        35                  40                  45

Ser Leu Arg Lys His Lys Asp Leu Tyr Arg Pro Leu Ser Leu Asp Asp
    50                  55                  60

Ser Asp Ser Leu Gly Asp Ser Met
65                  70

(2) INFORMATION FOR SEQ ID NO: 19:

(i) SEQUENCE CHARACTERISTICS:
(A) LENGTH: 422 amino acids
(B) TYPE: amino acid
(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO: 19:

Met Leu Glu Leu Ile Glu Val Asn Gly Thr Pro Gly Ser Gln Leu Ser
1               5                   10                  15

Thr Pro Arg Ser Gly Lys Ser Pro Ser Pro Ser Pro Thr Ser Pro Gly
            20                  25                  30

Ser Leu Arg Lys Gln Arg Ile Ser Gln His Gly Gly Ser Ser Thr Ser
        35                  40                  45

Leu Ser Ser Thr Lys Val Cys Ser Ser Met Asp Glu Asn Asp Gly Pro
    50                  55                  60

Gly Glu Glu Glu Ser Asp Glu Gly Phe Gln Ile Pro Ala Thr Ile Thr
65                  70                  75                  80

Glu Arg Tyr Lys Val Gly Arg Thr Ile Gly Asp Gly Asn Phe Ala Val
                85                  90                  95

Val Lys Glu Cys Ile Glu Arg Ser Thr Ala Arg Glu Tyr Ala Leu Lys
            100                 105                 110

Ile Ile Lys Lys Ser Lys Cys Arg Gly Lys Glu His Met Ile Gln Asn
        115                 120                 125

Glu Val Ser Ile Leu Arg Arg Val Lys His Pro Asn Ile Val Leu Leu
    130                 135                 140

Ile Glu Glu Met Asp Val Pro Thr Glu Leu Tyr Leu Val Met Glu Leu
145                 150                 155                 160

Val Lys Gly Gly Asp Leu Phe Asp Ala Ile Thr Ser Thr Ser Lys Tyr
                165                 170                 175

Thr Glu Arg Asp Ala Ser Gly Met Leu Tyr Asn Leu Ala Ser Ala Ile
            180                 185                 190

Lys Tyr Leu His Ser Leu Asn Ile Val His Arg Asp Ile Lys Pro Glu
        195                 200                 205

Asn Leu Leu Val Tyr Glu His Gln Asp Gly Ser Lys Ser Leu Lys Leu
    210                 215                 220

Gly Asp Phe Gly Leu Ala Thr Ile Val Asp Gly Pro Leu Tyr Thr Val
225                 230                 235                 240

Cys Gly Thr Pro Thr Tyr Val Ala Pro Glu Ile Ile Ala Glu Thr Gly
                245                 250                 255

Tyr Gly Leu Lys Val Asp Ile Trp Ala Ala Gly Val Ile Thr Tyr Ile
            260                 265                 270

Leu Leu Cys Gly Phe Pro Pro Phe Arg Gly Ser Gly Asp Asp Gln Glu
        275                 280                 285

Val Leu Phe Asp Gln Ile Leu Met Gly Gln Val Asp Phe Pro Ser Pro
    290                 295                 300

Tyr Trp Asp Asn Val Ser Asp Ser Ala Lys Gln Leu Ile Asn Met Met
305                 310                 315                 320

Leu Leu Val Asn Val Asp Gln Arg Phe Ser Ala Val Gln Val Leu Glu
                325                 330                 335

His Pro Trp Val Asn Asp Asp Gly Leu Pro Glu Asn Glu His Gln Leu
            340                 345                 350

Ser Val Ala Gly Lys Ile Lys Lys His Phe Asn Thr Gly Pro Lys Pro

        355                 360                 365

Ser Ser Thr Ala Ala Gly Val Ser Val Ile Ala Leu Asp His Gly Phe
    370                 375                 380

Thr Ile Lys Arg Ser Gly Ser Leu Asp Tyr Tyr Gln Gln Pro Gly Met
385                 390                 395                 400

Tyr Trp Ile Arg Pro Pro Leu Leu Val Arg Arg Gly Arg Phe Ser Asp
                405                 410                 415

Glu Asp Ala Thr Arg Met
            420

(2) INFORMATION FOR SEQ ID NO: 20:

(i) SEQUENCE CHARACTERISTICS:
(A) LENGTH: 502 amino acids
(B) TYPE: amino acid
(C) STRANDEDNESS: unknown
(D) TOPOLOGY: unknown

(ii) MOLECULE TYPE: protein

(iii) HYPOTHETICAL: NO

(xi) SEQUENCE DESCRIPTION: SEQ ID NO: 20:

Met Ser Cys Ala Gly Asn Asp Gln Ala Ala Ala Ser Gly Ser Ser Ser
1               5                   10                  15

Gly Ser Gly Gly Ile Phe Arg Ser Pro Ala Ala Lys Met Leu Lys Val
            20                  25                  30

Thr Val Pro Ser Cys Pro Ser Ser Pro Cys Ser Ser Val Thr Ser Ser
        35                  40                  45

Thr Glu Asn Leu Val Pro Asp Tyr Trp Ile Asp Gly Ser Lys Arg Asp
    50                  55                  60

Pro Leu Ser Asp Phe Phe Glu Val Glu Ser Glu Leu Gly Arg Gly Ala
65                  70                  75                  80

Thr Ser Ile Val Tyr Arg Cys Lys Gln Lys Gly Thr Gln Lys Pro Tyr
                85                  90                  95

Ala Leu Lys Val Leu Lys Lys Thr Val Asp Lys Lys Ile Val Arg Thr
            100                 105                 110

Glu Ile Gly Val Leu Ile Arg Leu Ser His Pro Asn Ile Ile Lys Leu
        115                 120                 125

Lys Glu Ile Phe Glu Thr Pro Thr Glu Ile Ser Leu Val Leu Glu Leu
    130                 135                 140

Val Thr Gly Gly Glu Leu Phe Asp Arg Ile Val Glu Lys Gly Tyr Tyr
145                 150                 155                 160

Ser Glu Arg Asp Ala Ala Asp Ala Val Lys Gln Ile Leu Glu Ala Val
                165                 170                 175

Ala Tyr Leu His Glu Asn Gly Ile Val His Arg Asp Leu Lys Pro Glu
            180                 185                 190

Asn Leu Leu Tyr Ala Thr Pro Ala Pro Asp Ala Pro Leu Lys Ile Ala
        195                 200                 205

Asp Phe Gly Leu Ser Lys Ile Val Glu His Gln Val Leu Met Lys Thr
    210                 215                 220

Val Cys Gly Thr Pro Gly Tyr Cys Ala Pro Glu Ile Leu Arg Gly Cys
225                 230                 235                 240

Ala Tyr Gly Pro Glu Val Asp Met Trp Ser Val Gly Ile Ile Thr Tyr
                245                 250                 255

Ile Leu Leu Cys Gly Phe Glu Pro Phe Tyr Asp Glu Arg Gly Asp Gln
            260                 265                 270

Phe Met Phe Arg Arg Ile Leu Asn Cys Glu Tyr Tyr Phe Ile Ser Pro
        275                 280                 285

Trp Trp Asp Glu Val Ser Leu Asn Ala Lys Asp Leu Val Lys Lys Leu
    290                 295                 300

Ile Val Leu Asp Pro Lys Lys Arg Leu Thr Thr Phe Gln Ala Leu Gln
305                 310                 315                 320

His Pro Trp Val Thr Gly Lys Ala Ala Asn Phe Val His Met Asp Thr
                325                 330                 335

Ala Gln Lys Lys Leu Gln Glu Phe Asn Ala Arg Arg Lys Leu Lys Ala
            340                 345                 350

Ala Val Lys Ala Val Val Ala Ser Ser Arg Leu Gly Ser Ala Ser Ser
        355                 360                 365

Ser His Thr Asn Ile Gln Glu Ser Asn Lys Ala Ser Ser Glu Ala Gln
    370                 375                 380

Pro Ala Gln Asp Gly Lys Asp Lys Thr Asp Pro Leu Glu Asn Lys Met
385                 390                 395                 400

Gln Ala Gly Asp His Glu Ala Ala Lys Ala Ala Ala Asp Glu Thr Met
                405                 410                 415

Lys Leu Gln Ser Glu Glu Val Glu Glu Glu Gln Gly Val Lys Glu Glu
            420                 425                 430

Glu Glu Glu Glu Glu Glu Glu Glu Glu Thr Ser Arg Met Val Pro Gln
        435                 440                 445

Glu Pro Glu Asp Arg Leu Glu Thr Asp Asp Gln Glu Met Lys Arg Asn

    450                 455                 460

Ser Glu Glu Thr Leu Lys Ser Val Glu Glu Glu Met Asp Pro Lys Ala
465                 470                 475                 480

Glu Glu Glu Ala Ala Ala Val Gly Leu Gly Val Pro Pro Gln Gln Asp
                485                 490                 495

Ala Ile Leu Pro Glu Tyr
            500